# EUROPEAN PATENT SPECIFICATION

(11) **EP 2 989 215 B1**
(45) Date of publication and mention of the grant of the patent: **04.09.2019**
(21) Application number: 14788247.6
(22) Date of filing: 25.04.2014
(51) Int. Cl.: C12Q 1/682, C12Q 1/6806

(54) **MULTIPLEXED ANALYSIS OF TARGET NUCLEIC ACIDS**
MULTIPLEX-ANALYSE VON ZIELNUKLEINSÄUREN
ANALYSE MULTIPLEXÉE D'ACIDES NUCLÉIQUES CIBLES

(30) Priority: 25.04.2013 US 201361816070 P; 06.02.2014 US 201461936826 P
(43) Date of publication of application: 02.03.2016
(73) Proprietor: Firefly Bioworks, Inc., Cambridge, MA 02139 (US)
(72) Inventor: PREGIBON, Daniel, C., Somerville, MA 02144 (US); STONER, Isaac, Cambridge, MA 02139 (US); FUSCO, Anthony, Westwood, MA 02090 (US); TACKETT, Michael, R., Cambridge, MA 02140 (US)
(74) Representative: Carpmaels & Ransford LLP
(86) International application number: PCT/US2014/035578
(87) International publication number: WO 2014/176575

(56) References cited:
- EP-A2- 0 328 829
- EP-A2- 1 184 466
- WO-A1-2006/108473
- WO-A1-2008/045158
- WO-A2-01/83814
- US-A- 6 001 571
- US-A1- 2009 156 425
- US-A1- 2012 100 526
- US-B1- 6 709 813
- GAO HUAFANG ET AL: "Comparison of different methods for preparing single stranded DNA for oligonucleotide microarray", ANALYTICAL LETTERS, TAYLOR & FRANCIS INC, US, vol. 36, no. 13, 1 October 2003 (2003-10-01), pages 2849-2863, XP008092152, ISSN: 0003-2719, DOI: 10.1081/AL-120025260

## Description

### Background

Nucleic acids are becoming increasingly important for diagnostic and therapeutic use. For example, early and accurate detection of various nucleic acid biomarkers or genomic mutations and imbalance present in diseased cells can have important clinical implications. Multiplexing technologies are powerful tools in analyzing nucleic acids, especially in both the laboratory and diagnostic setting. However, many of these methods are limited by low sensitivity, cross-reactivity of nonspecific targets, or assay/instrumentation complexity.

EP0328829, WO2008/045158 and EP1184466 each discloses a method comprising capture of target nucleic acids by hybridization to immobilized probes, elution and amplification of the target, followed by probe-mediated capture of the products on a second solid support, for instance for detection.

### Summary

The present invention addresses many of the limitations faced by the conventional multiplexing technologies that exist in the prior art and provides more robust, reliable and efficient methods and systems for analyzing nucleic acids including, but not limited to, multiple microRNAs, mRNAs, long-noncoding RNAs (lncRNAs), genomic DNAs, and synthetic RNAs such as siRNA in a single sample. As described herein, methods and compositions described herein are particularly effective in capturing, analyzing or quantifying low abundance target nucleic acids in a biological sample. Methods and compositions described herein may be used to analyze single target nucleic acid or multiple target nucleic acids simultaneously.

Thus, in one aspect, the present invention provides methods of detecting target nucleic acid, comprising steps of: a) contacting a sample with a first set of particles bearing a plurality of capturing probes, each comprising at least one target capturing sequence, under conditions that permit the capturing probes to capture one or more target nucleic acids in the sample; b) amplifying the captured one or more target nucleic acids in a reaction mixture comprising a detectable entity such that the amplified one or more target nucleic acids are labeled with the detectable entity; c) incubating amplification product with a second set of particles bearing a plurality of re-capturing probes such that the amplified one or more target nucleic acids are re-captured by the plurality of the re-capturing probes, wherein the first set and second set of particles are identical; and d) detecting signal generated by a detectable entity associated with the re-captured amplified one or more target nucleic acids, wherein the presence and/or abundance of the detectable signal indicates the presence and/or abundance of the one or more target nucleic acids in the sample.

In some embodiments, each of the capturing probes comprises one target capturing sequence and binds specifically to one distinct target nucleic acid. In some embodiments, each of the capturing probes comprises multiple distinct target capturing sequences and binds to multiple distinct target nucleic acids. In some embodiments, each of the re-capturing probes comprises one target capturing sequence and binds specifically to one distinct target nucleic acid. In some embodiments, each of the re-capturing probes comprises multiple distinct target capturing sequences and binds multiple distinct target nucleic acids.

In some embodiments, the particles are made of a material selected from the group consisting of hydrogel, glass, photoresist, silica, polystyrene, polyethylene glycol, agarose, chitosan, alginate, PLGA, optical fiber, cellulose, and combination thereof. In some embodiments, the material is hydrogel. In some embodiments, the capturing and re-capturing probes are embedded throughout one or more probe regions of the particle. In some embodiments, the particle further comprises one or more encoding regions and wherein the one or more encoding regions bear detectable moieties that give the identity of the capturing or re-capturing probes.

In some embodiments, the one or more target nucleic acids are microRNAs, mRNAs, non-coding transcripts, genomic DNA, cDNAs, siRNAs, DNA/RNA chimera, or combination thereof. In some embodiments, the probe is DNA, RNA, DNA/RNA chimera, or combination thereof. In some embodiments, the probe specific to the target nucleic acid comprises a target capture sequence that is substantially complementary to the target nucleic acid.

In some embodiments, the method further comprises a step of coupling one or more adapters to the captured target nucleic acid. In some embodiments, the one or more adapters are universal adapters. In some embodiments, the one or more adapters are coupled to the target nucleic acid at the 3'-terminus, the 5'-terminus, or both the 3'-terminus and 5'-terminus. In some embodiments, the one or more adapters are DNA, RNA, DNA/RNA chimera, or combination thereof.

In some embodiments, the captured target nucleic acid is first digested by a nuclease or restriction enzyme to remove single-stranded 5' and or 3' regions prior to the coupling of the one or more adapters. In some embodiments, each of the capturing probes further comprises sequences complementary to the one or more adapters. In some embodiments, the sequences complementary to the one or more adapters are adjacent to the target capture sequence. In some embodiments, the one or more adapters are coupled to the target nucleic acid via ligation. In some embodiments, the one or more adapters contain sequences substantially complementary to PCR primer sequences. In some embodiments, the one or more adapters contain sequences substantially similar to PCR primer sequences.

In some embodiments, the ligation is performed by a DNA or RNA ligase enzyme. In some embodiments, the one or more adapters comprise sequences specifically designed to serve as sites for polymerase chain reaction priming, reverse transcription, or modification by other DNA-modifying or RNA-modifying enzymes.

In some embodiments, the step of amplifying the captured target nucleic acid comprises performing a polymerase chain reaction (PCR). In some embodiments, the PCR reaction uses polymerase enzyme selected from Taq, Bst, and/or Phi29. In some embodiments, the captured target is reverse transcribed prior to amplification. In some embodiments, reverse transcription is catalyzed by a polymerase enzyme with reverse transcriptase activity. In some embodiments, the polymerase enzyme is Pyrophage or TtH. In some embodiments, reverse transcription is catalyzed by one enzyme and PCR amplification is carried out by a second enzyme.

In some embodiments, the step of amplifying the captured target nucleic acid is performed isothermally. In some embodiments, the target nucleic acid and/or the one or more adapters are circularized via ligation or enzymatic polymerization.

In some embodiments, the PCR is performed with a single primer pair. In some embodiments, the PCR is performed with one primer. In some embodiments, the PCR is performed with primers attached to the particle. In some embodiments, the PCR is performed using a combination of universal, specific, or poly(A) primers.

In some embodiments, the detectable entity is selected from the group consisting of fluorophores, dyes, biotin, radioisotopes, antibodies, aptamers, polypeptides, quantum dots, chromophores, or signal-generating enzymes. In some embodiments, the detectable entity is provided in the reaction mixture as labeled primer, labeled dNTPs and/or intercalating dye. In some embodiments, the detectable entity is associated with the forward primer. In some embodiments, the detectable entity is associated with the reverse primer. In some embodiments, the detectable entity is associated with multiple primers.

In some embodiments, the captured one or more target nucleic acids are separated from the capturing probes prior to amplification. In some embodiments, the captured one or more target nucleic acids are separated from the capturing probes by denaturation using heat, chemical denaturants, or a helicase enzyme.

In some embodiments, the substrate is present during the time of amplification. In some embodiments, the step of amplifying the captured one or more target nucleic acids is performed using a single primer. In some embodiments, the step of amplifying the captured one or more target nucleic acids is performed using less than 5 (e.g., less than 4, 3, or 2) primer pairs.

In some embodiments, the PCR is biased such that a substantial fraction of the amplified one or more target nucleic acids is single-stranded. In some embodiments, the PCR is biased towards single-stranded amplified target nucleic acid through designing a forward primer with a significantly lower annealing temperature than a reverse primer and first thermocycling at the lower annealing temperature and then thermocycling at the higher annealing temperature. In some embodiments, the PCR is biased towards single-stranded amplified target nucleic acid through adding the forward primer at a concentration such that it is exhausted during the PCR reaction. In some embodiments, the ratio between the forward primer and the reverse primer is less than 1:2, 1:3, 1:4, 1:5, or 1:10. In some embodiments, the ratio between the forward primer and the reverse primer is less than 1:2. In some embodiments, the PCR is biased towards single-stranded amplified target nucleic acid through designing a reverse primer with a significantly lower annealing temperature than a forward primer and first thermocycling at the lower annealing temperature and then thermocycling at the higher annealing temperature. In some embodiments, the PCR is biased towards single-stranded amplified target nucleic acid through adding the reverse primer at a concentration such that it is exhausted during the PCR reaction. In some embodiments, the ratio between the reverse primer and the forward primer is less than 1:2, 1:3, 1:4, 1:5, or 1:10. In some embodiments, the ratio between the reverse primer and the forward primer is less than 1:2.

In some embodiments, the amplification product and the plurality of re-capturing probes are incubated under stringent hybridization condition.

In some embodiments, the substrate is rinsed between steps to remove unbound probes, target nucleic acids and/or adapters.

In some embodiments, the capturing and re-capturing probes contain one or more mismatch bases against target nucleic acid.

In some embodiments, the conditions are tuned in order to give stringent capture by controlling: temperature, time, monovalent salt concentration, divalent salt concentration, dNTP concentration, or the addition of DMSO, formamide, polyethylene glycol, 2-pyrrolidone, propylene glycol, or other agents that alter the kinetics of DNA duplex formation.

In some embodiments, the sample is a biological sample. In some embodiments, the biological sample is a preparation of isolated DNA or RNA, protease tissue digest, cell lysate, serum, plasma, whole blood, urine, stool, saliva, cord blood, chorionic villus sample, chorionic villus sample culture, amniotic fluid, amniotic fluid culture, transcervical lavage fluid, and combination thereof.

In some embodiments, the signal generated by detectable entity is detected by a flow cytometer, or array scanner. In some embodiments, the signal is quantified.

In some embodiments, the one or more capturing probes comprises multiple capturing probes specific to multiple target nucleic acids. In some embodiments, the multiple probes are associated with multiple particles, with each particle comprising probes specific to a same target nucleic acid. In some embodiments, each particle is encoded to provide identity of the specific probes thereon. In some embodiments, each particle is encoded through incorporation of one or more fluorophores with known spectral characteristics.

In some embodiments, the re-capturing of amplified one or more target nucleic acids are performed under substantially more stringent conditions than the capturing step.

In some embodiments, the reaction mixture comprises a single primer set used to amplify multiple distinct target nucleic acids.

In some embodiments, the reaction mixture comprises multiple primer sets used to amplify multiple distinct target nucleic acids.

In some embodiments, each target nucleic acid is present at low abundance in the sample.

In some embodiments, each target nucleic acid represents less than 1% of total nucleic acids in the biological sample. In some embodiments, each target nucleic acid represents less than 0.1% of total nucleic acids in the biological sample. In some embodiments, each target nucleic acid represents less than 1 out of a million of total nucleic acids in the biological sample. In some embodiments, each target nucleic acid represents less than 1 out of 10 million of total nucleic acids in the biological sample.

In another aspect, the present disclosure provides methods of detecting target nucleic acid, comprising steps of: a) contacting a sample comprising one or more target nucleic acids with a first set of particles bearing a plurality of capturing probes, each comprising at least one target capturing sequence, under conditions that permit the plurality of capturing probes to capture one or more target nucleic acids in the sample; b) amplifying the captured one or more target nucleic acids in a reaction mixture comprising a detectable entity such that the amplified one or more target nucleic acids are labeled with the detectable entity; and c) incubating amplification product with the original set of particles or a second set of particles bearing a plurality of re-capturing probes such that the amplified one or more target nucleic acids are re-captured by the plurality of the re-capturing probes; wherein each particle has one or more probe regions bearing the plurality of capturing or re-capturing probes and one or more encoding regions bearing detectable moieties that give the identity of the capturing or re-capturing probes thereon; and wherein the presence and/or abundance of the detectable signal generated by detectable entity associated with the re-captured amplified one or more target nucleic acids on the second set of particles indicates the presence and/or abundance of the one or more target nucleic acids in the sample.

In some embodiments, the method comprises a step of scanning the second set of particles by a flow-through device to detect the presence and/or abundance of the detectable signal associated with the re-captured amplified one or more target nucleic acids and the detectable moieties associated with the one or more encoding regions of the particles.

In some embodiments, the first set of particles comprise distinct particles bearing distinct capturing probes. In some embodiments, each particle bears a plurality of identical capturing probes. In some embodiments, the second set of particles comprise distinct particles bearing distinct re-capturing probes. In some embodiments, each particle bears a plurality of identical re-capturing probes. In some embodiments, the first set and second set of particles are identical. In some embodiments, the first and second set of particles are the same set. In some embodiments, the particles are made of a material selected from the group consisting of hydrogel, glass, photoresists, silica, polystyrene, polyethylene glycol, agarose, chitosan, alginate, PLGA, optical fiber, cellulose, and combination thereof. In some embodiments, the particles are hydrogel particles. In some embodiments, the particles have greater than about 1 µm up to about 450 µm in at least one dimension.

In some embodiments, the capturing or re-capturing probes are embedded throughout one or more spatially defined probe regions of the particle. In some embodiments, the particle further comprises one or more encoding regions and wherein the one or more encoding regions bear detectable moieties that give the identity of the capturing or re-capturing probes.

In some embodiments, the one or more target nucleic acids are microRNAs, mRNAs, non-coding transcripts, genomic DNA, cDNAs, siRNAs, DNA/RNA chimera, or combination thereof. In some embodiments, the probe is DNA, RNA, DNA/RNA chimera, or combination thereof. In some embodiments, the probe specific to the target nucleic acid comprises a target capture sequence that is substantially complementary to the target nucleic acid.

In some embodiments, the method further comprises a step of coupling one or more adapters to the captured one or more target nucleic acids. In some embodiments, the one or more adapters are universal adapters. In some embodiments, the one or more adapters are coupled to the target nucleic acid at the 3'-terminus, the 5'-terminus, or both the 3'-terminus and 5'-terminus. In some embodiments, the one or more adapters are DNA, RNA, DNA/RNA chimera, or combination thereof. In some embodiments, the captured target nucleic acid is first digested by a nuclease or restriction enzyme to remove single-stranded 5' and or 3' regions prior to the coupling of the one or more adapters.

In some embodiments, each of the capturing probes further comprises sequences complementary to the one or more adapters. In some embodiments, the sequences complementary to the one or more adapters are adjacent to the target capture sequence. In some embodiments, the one or more adapters are coupled to the target nucleic acid via ligation. In some embodiments, the ligation is performed by a DNA or RNA ligase enzyme. In some embodiments, the one or more adapters comprise sequences specifically designed to serve as sites for polymerase chain reaction priming, reverse transcription, or modification by other DNA-modifying or RNA-modifying enzymes.

In some embodiments, the step of amplifying the captured target nucleic acid comprises performing a polymerase chain reaction (PCR). In some embodiments, the captured one or more target nucleic acids are amplified in the presence of the particles. In some embodiments, the captured one or more target nucleic acids are first separated from the particles prior to amplification. In some embodiments, the captured target is reverse transcribed prior to amplification.

In some embodiments, the reaction mixture for amplification comprises a polymerase enzyme with reverse transcriptase activity. In some embodiments, the polymerase enzyme Pyrophage or TtH. In some embodiments, the reaction mixture for amplification comprises a reverse transcriptase and a separate polymerase enzyme. In some embodiments, the polymerase enzyme is selected from Taq, Bst, and/or Phi29.

In some embodiments, the step of amplifying the captured target nucleic acid is performed isothermally. In some embodiments, the target nucleic acid and/or the one or more adapters are circularized via ligation or enzymatic polymerization.

In some embodiments, the PCR is performed with a single primer set. In some embodiments, the PCR is performed with one primer. In some embodiments, the PCR is performed with primers attached to the substrate. In some embodiments, the PCR is performed using a combination of universal, specific, or poly(A) primers.

In some embodiments, the detectable entity is selected from the group consisting of fluorophores, dye, biotin, radioisotopes, antibodies, aptamers, polypeptides, quantum dots, chromophores. In some embodiments, the detectable entity is provided in the reaction mixture as labeled primer, labeled dNTPs and/or intercalating dye.

In some embodiments, the captured one or more target nucleic acids are separated from the capturing probes prior to amplification. In some embodiments, the captured one or more target nucleic acids are separated from the capturing probes by denaturation using heat, chemical denaturants, or a helicase enzyme.

In some embodiments, the particle is present during the time of amplification.

In some embodiments, the step of amplifying the captured one or more target nucleic acids is performed using a single primer. In some embodiments, the step of amplifying the captured one or more target nucleic acids is performed using less than 5 primer pairs.

In some embodiments, the PCR is biased such that a substantial fraction of the amplified one or more target nucleic acids is single-stranded. In some embodiments, the PCR is biased towards single-stranded amplified target nucleic acid through designing a forward primer with a significantly lower annealing temperature than a reverse primer and first thermocycling at the lower annealing temperature and then thermocycling at the higher annealing temperature. In some embodiments, the PCR is biased towards single-stranded amplified target nucleic acid through adding the forward primer at a concentration such that it is exhausted during the PCR reaction. In some embodiments, the ratio between the forward primer and the reverse primer is less than 1:2.

In some embodiments, the amplification product and the plurality of re-capturing probes are incubated under stringent hybridization condition.

In some embodiments, the particles are rinsed between steps to remove unbound probes, target nucleic acids and/or adapters.

In some embodiments, the capturing or re-capturing probes contain one or more mismatch bases against target nucleic acid.

In some embodiments, the conditions are tuned in order to give stringent capture by controlling: temperature, time, monovalent salt concentration, divalent salt concentration, dNTP concentration, or the addition of DMSO, formamide, polyethylene glycol, 2-pyrrolidone, or other agents that alter the kinetics of DNA duplex formation.

In some embodiments, the sample is a biological sample. In some embodiments, the biological sample is a preparation of isolated DNA or RNA, protease tissue digest, cell lysate, serum, plasma, whole blood, urine, stool, saliva, cord blood, chorionic villus sample, chorionic villus sample culture, amniotic fluid, amniotic fluid culture, transcervical lavage fluid, and combination thereof.

In some embodiments, the signal generated by detectable entity is detected by a flow cytometer, or array scanner. In some embodiments, the flow-through device is a flow cytometer or array scanner. In some embodiments, the signal is quantified.

In some embodiments, the one or more capturing probes comprise multiple capturing probes specific to multiple target nucleic acids. In some embodiments, the multiple probes are associated with multiple particles, with each particle comprising probes specific to same target nucleic acid. In some embodiments, the each particle is encoded to provide identity of the specific probes thereon. In some embodiments, the each particle is encoded through incorporation of one or more fluorophores with known spectral characteristics. In some embodiments, the multiple capturing probes are located on multiple distinct regions of a planar substrate. In some embodiments, the re-capturing of amplified one or more target nucleic acids are performed under substantially more stringent conditions than the capturing step.

In some embodiments, the reaction mixture comprises a single primer set used to amplify multiple distinct target nucleic acids. In some embodiments, the reaction mixture comprises multiple primer sets used to amplify multiple distinct target nucleic acids. In some embodiments, each target nucleic acid is present at low abundance in the sample. In some embodiments, each target nucleic acid represents less than 1% of total nucleic acids in the biological sample. In some embodiments, each target nucleic acid represents less than 0.1% of total nucleic acids in the biological sample. In some embodiments, each target nucleic acid represents less than 1 out of a million of total nucleic acids in the biological sample. In some embodiments, each target nucleic acid represents less than 1 out of 10 million of total nucleic acids in the biological sample.

In another aspect, the present disclosure provides diagnostic methods comprising a step of detecting one or more target nucleic acids according to any one of the preceding methods.

In another aspect, the present disclosure provides kits for detecting target nucleic acid. In some aspects, the disclosure provides a kit for detecting target nucleic acid, comprising: particles comprising one or more probe regions bearing probes and one or more coding regions bearing detectable moieties that give the identity of the probes thereon, wherein the probes comprise target capturing sequence; a hyrbridization buffer with pre-determined ionic strength, buffered pH, and denaturing reagent (e.g., formamide and/or 2-pyrrolidone); a labeling buffer comprising one or more oligonucleotide adapters specifically designed to serve as sites for polymerase chain reaction priming and/or reverse transcription; and a PCR buffer containing primers, dNTPs, and reaction reagents for amplification of captured targets. In some embodiments, the kit further comprises a reverse transcriptase and a polymerase. In some embodiments, the kit further comprise a polymerase enzyme that has reverse transcriptase activity. In some embodiments, the kit further comprises a ligase enzyme.

As used in this application, the terms "about" and "approximately" are used as equivalents. Any numerals used in this application with or without about/approximately are meant to cover any normal fluctuations appreciated by one of ordinary skill in the relevant art.

Other features, objects, and advantages of the present invention are apparent in the detailed description that follows. It should be understood, however, that the detailed description, while indicating embodiments of the present invention, is given by way of illustration only, not limitation. Various changes and modifications within the scope of the invention will become apparent to those skilled in the art from the detailed description.

### Brief Description of the Figures

The drawings are for illustration purposes only, not for limitation.
**Figure 1** illustrates an exemplary schematic for specific capture, modification, and universal amplification of nucleic acid targets. Targets are captured with target-specific probes in encoded hydrogel particles, adapter sequences are ligated to the end, and sequences in the adapter site are used for priming in PCR-based amplification. Fluorescent amplicons are then captured on the particles for quantification.
**Figure 2** illustrates an exemplary schematic for specific capturing, labeling, amplification, recapturing, scanning, and analyzing nucleic acid targets, (a) Exemplary probes before the process illustrated in (c). (b) Exemplary probes after the process illustrated in (c). (c) Targets are captured with target-specific probes in encoded hydrogel particles, adapter sequences are ligated to the end, and sequences in the adapter site are used for priming in PCR-based amplification. Fluorescent amplicons are then captured on the particles for quantification.
**Figure 3** shows an exemplary graph demonstrating that the limit of detection of this multiplexed PCR-coupled hybridization assay may be as low as 100 molecules per sample at the cycling conditions used.
**Figure 4** show an exemplary graph demonstrating the signal for three detected targets and three undetected targets with increasing cycle number. This shows that the sensitivity and dynamic range covered by this multiplexed PCR-coupled hybridization assay can be shifted as needed.
**Figure 5** shows an exemplary comparison of microRNA profiles of RNA isolated from three tissue types: lung, brain, and placenta. The results of the multiplexed PCR-coupled hybridization assay, referred to as Firefly HS", were directly compared to profiles resulting from RNA-Seq on the Illumina platform, Taqman qPCR (TLDA card format), and microarray analysis. Triplicate measurements demonstrate robust profiles that cluster well between the different analysis methods used. The Pearson correlations between each method are shown.
**Figure 6** shows an exemplary comparison of total RNA isolated from brain tissue assayed with the PCR-coupled hybridization assay across two logs of total RNA input.
**Figure 7** shows an exemplary graph of microRNA profiling from serum RNA. RNA that was isolated from human serum was assayed in triplicate for 30 microRNA targets with the novel PCR-coupled assay.
**Figure 8** shows an exemplary graph of microRNA profiling from serum treated with a buffer containing proteinase K, a surfactant, and a chaotropic salt that served to disrupt RNA-associated proteins and exosomes as well as inhibit the activity of RNA-degrading enzymes.
**Figure 9** illustrates an exemplary schematic for the detection of RNA with capture, modification, and amplification using a poly(T) primer. A single adapter is ligated to the 5' end of the mRNA species, and universal amplification is performed with a primer sequence within the adapter region and one that contains a poly(T) region to prime against the poly(A) mRNA tail.
**Figure 10** illustrates an exemplary schematic of the labeling and amplification of targets with target end-specific probes. Probes are complementary to the terminal sequences of the target species, thus aligning it for ligation of adapters on both ends.
**Figure 11** illustrates an exemplary schematic of nuclease digestion for detection of sequences internal to a nucleic acid target. After hybridization, single stranded regions of targets are digested with a nuclease. Truncated targets are then labeled and amplified for detection or isolation.

### Definitions

In order for the present invention to be more readily understood, certain terms are first defined below. Additional definitions for the following terms and other terms are set forth throughout the specification.

***"Adjacent":*** As used herein, the term "adjacent" means "next to," "contiguous," "adjoining," "abutting" or having a common boundary.

***"Analyte":*** As used herein, the term "analyte" broadly refers to any substance to be analyzed, detected, measured, or quantified. Examples of analytes include, but are not limited to, proteins, peptides, hormones, haptens, antigens, antibodies, receptors, enzymes, nucleic acids, and combinations thereof.

***"Associated":*** As used herein, the terms "associated", "conjugated", "linked", "attached", "complexed", and "tethered," and grammatical equivalents, typically refer to two or more moieties connected with one another, either directly or indirectly (e.g., via one or more additional moieties that serve as a linking agent), to form a structure that is sufficiently stable so that the moieties remain connected under the conditions in which the structure is used, *e.g.,* physiological conditions. In some embodiments, the moieties are attached to one another by one or more covalent bonds. In some embodiments, the moieties are attached to one another by a mechanism that involves specific (but non-covalent) binding (*e.g.* streptavidin/avidin interactions, antibody/antigen interactions, *etc.*)*.* Alternatively or additionally, a sufficient number of weaker interactions (non-covalent) can provide sufficient stability for moieties to remain connected. Exemplary non-covalent interactions include, but are not limited to, affinity interactions, metal coordination, physical adsorption, host-guest interactions, hydrophobic interactions, pi stacking interactions, hydrogen bonding interactions, van der Waals interactions, magnetic interactions, electrostatic interactions, dipole-dipole interactions, *etc.*

***"Complement":*** As used herein, the terms "complement," "complementary" and "complementarity," refer to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence. Certain bases not commonly found in natural nucleic acids may be included in the complementary nucleic acids including, but not limited to, inosine, 7-deazaguanine, Locked Nucleic Acids (LNA), and Peptide Nucleic Acids (PNA). Complementary need not be perfect; stable duplexes may contain mismatched base pairs, degenerative, or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

***"Contemporaneous"*** and ***"non-contemporaneous":*** As used herein, the terms "contemporaneous," "contemporaneously," or grammatical equivalents, mean that multiple events occur or happen at the same time without a detectable or identifiable sequential order. As used herein, the terms "non-contemporaneous," "non-contemporaneously," or grammatical equivalents, mean that multiple events occur or happen in a detectable or identifiable sequential order.

***"Crude":*** As used herein, the term "crude," when used in connection with a biological sample, refers to a sample which is in a substantially unrefined state. For example, a crude sample can be cell lysates or biopsy tissue sample. A crude sample may exist in solution or as a dry preparation.

***"Encoding region," "coding region," or "barcoded region*** ": As used herein, the terms "encoding region," "coding region," "barcoded region", or grammatical equivalents, refer to a region on an object or substrate (e.g., particle) that can be used to identify the object or substrate (e.g., particle). These terms may be used inter-changeably. Typically, an encoding region of an object bears graphical and/or optical features associated with the identity of the object. Such graphical and/or optical features are also referred to as signature features of the object. In some embodiments, an encoding region of an object bears spatially patterned features (e.g., stripes with various shapes and/or dimensions, or a series of holes with various sizes) that give rise to variable fluorescent intensities (of one or multiple wavelengths). In some embodiments, an encoding region of an object bears various type and/or amount of fluorophores or other detectable moieties, in various spatial patterns, that give rise to variable fluorescent signals (e.g., different colors and/or intensities) in various patterns.

***"Functionalization:*** As used herein, the term "functionalization" refers to any process of modifying a material by bringing physical, chemical or biological characteristics different from the ones originally found on the material. Typically, functionalization involves introducing functional groups to the material. As used herein, functional groups are specific groups of atoms within molecules that are responsible for the characteristic chemical reactions of those molecules. As used herein, functional groups include both chemical (e.g., ester, carboxylate, alkyl) and biological groups (e.g., oligonucleotide adapter, or linker sequences).

***"Hybridize":*** As used herein, the term "hybridize" or "hybridization" refers to a process where two complementary nucleic acid strands anneal to each other under appropriately stringent conditions. Oligonucleotides or probes suitable for hybridizations typically contain 10-100 nucleotides in length (e.g., 18- 50, 12-70, 10-30, 10-24, 18-36 nucleotides in length). Nucleic acid hybridization techniques are well known in the art. See, e.g., Sambrook, et al., 1989, Molecular Cloning: A Laboratory Manual, Second Edition, Cold Spring Harbor Press, Plainview, N.Y. Those skilled in the art understand how to estimate and adjust the stringency of hybridization conditions such that sequences having at least a desired level of complementary will stably hybridize, while those having lower complementary will not. For examples of hybridization conditions and parameters, see, e.g., Sambrook, et al., 1989, Molecular Cloning: A Laboratory Manual, Second Edition, Cold Spring Harbor Press, Plainview, N.Y.; Ausubel, F. M. et al. 1994, Current Protocols in Molecular Biology. John Wiley & Sons, Secaucus, N.J.

***"Inert region ":*** As used herein, the terms "inert region," "inert spacer" or grammatical equivalents, when used in connection with a region on a substrate (e.g., particle), refer to a region that is not detectable above a pre-determined triggering threshold by a flow-through scanning device such as a flow cytometer. Typically, an inert region or spacer is a non-functionalized region. For example, an inert region is a region not loaded with probes or other detectable moieties.

***"Interrogate":*** As used herein, the terms "interrogate," "interrogating," "interrogation" or grammatical equivalents, refer to a process of characterizing or examining to obtain data.

***"Labeled":*** The terms "labeled" and "labeled with a detectable agent or moiety" are used herein interchangeably to specify that an entity (*e.g.,* a nucleic acid probe, antibody, *etc.*) can be visualized, for example following binding to another entity (*e.g.,* a nucleic acid, polypeptide, *etc.*)*.* The detectable agent or moiety may be selected such that it generates a signal which can be measured and whose intensity is related to (e.g., proportional to) the amount of bound entity. A wide variety of systems for labeling and/or detecting proteins and peptides are known in the art. Labeled proteins and peptides can be prepared by incorporation of, or conjugation to, a label that is detectable by spectroscopic, photochemical, biochemical, immunochemical, electrical, optical, chemical or other means. A label or labeling moiety may be directly detectable (*i.e*., it does not require any further reaction or manipulation to be detectable, *e.g.,* a fluorophore is directly detectable) or it may be indirectly detectable (*i.e.,* it is made detectable through reaction or binding with another entity that is detectable, e.g., a hapten is detectable by immunostaining after reaction with an appropriate antibody comprising a reporter such as a fluorophore). Suitable detectable agents include, but are not limited to, radionucleotides, fluorophores, chemiluminescent agents, microparticles, enzymes, colorimetric labels, magnetic labels, haptens, molecular beacons, aptamer beacons, and the like.

***"Monodisperse":*** As used herein, the terms *"monodisperse"* or *"monosized"* refer to a collection of objects that have substantially the same size and shape when in the context of particles, and substantially the same mass in the context of polymers. Conversely, a collection of objects that have an inconsistent size, shape and mass distribution are called polydisperse. Monodisperse particles are typically synthesized through the use of template-based synthesis.

***"Object" or "substrate":*** As used herein, the terms "object" and "substrate" are used interchangeably and refer to any discrete mass. An object or substrate can be a particle, bead, planar surface, phage, macromolecules, cell, micro-organism, and the like.

***"Particle":*** The term "particle," as used herein, refers to a discrete object. Such object can be of any shape or size. Composition of particles may vary, depending on applications and methods of synthesis. Suitable materials include, but are not limited to, plastics, ceramics, glass, polystyrene, methylstyrene, acrylic polymers, metal, paramagnetic materials, thoria sol, carbon graphited, titanium dioxide, latex or cross-linked dextrans such as Sepharose, cellulose, nylon, cross-linked micelles and teflon. In some embodiments, particles can be optically or magnetically detectable. In some embodiments, particles contain fluorescent or luminescent moieties, or other detectable moieties. In some embodiments, particles having a diameter of less than 1000 micrometers (um) are also referred to as microparticles. In some embodiments, particles having a diameter of less than 1000 nanometers (nm) are also referred to as nanoparticles.

***"Polynucleotide", "nucleic acid",*** or ***"oligonucleotide":*** The terms "polynucleotide", "nucleic acid", or "oligonucleotide" refer to a polymer of nucleotides. The terms "polynucleotide", "nucleic acid", and "oligonucleotide", may be used interchangeably. Typically, a polynucleotide comprises at least three nucleotides. DNAs and RNAs are polynucleotides. The polymer may include natural nucleosides (*i.e.,* adenosine, thymidine, guanosine, cytidine, uridine, deoxyadenosine, deoxythymidine, deoxyguanosine, and deoxycytidine), nucleoside analogs (*e.g*., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g*., methylated bases), intercalated bases, modified sugars (*e.g*., 2'-fluororibose, ribose, 2'-deoxyribose, arabinose, and hexose), or modified phosphate groups (*e.g*., phosphorothioates and 5'-N-phosphoramidite linkages).

***"Probe":*** As used herein, the term "probe" refers to a fragment of DNA or RNA of variable length (e.g., 3-1000 bases long), which is used to detect the presence of target nucleotide sequences that are complementary to the sequence in the probe. Typically, the probe hybridizes to single-stranded nucleic acid (DNA or RNA) whose base sequence allows probe-target base pairing due to complementarity between the probe and target.

***"Secondary Structure":*** As used herein, the term "secondary structure", when used in connection with a nucleic acid structure, refers to any structure formed by basepairing interactions within a single molecule or set of interacting molecules. Exemplary secondary structures include stem-loop or double helix.

***"Signal":*** As used herein, the term "signal" refers to a detectable and/or measurable entity. In certain embodiments, the signal is detectable by the human eye, *e.g.,* visible. For example, the signal could be or could relate to intensity and/or wavelength of color in the visible spectrum. Non-limiting examples of such signals include colored precipitates and colored soluble products resulting from a chemical reaction such as an enzymatic reaction. In certain embodiments, the signal is detectable using an apparatus. In some embodiments, the signal is generated from a fluorophore that emits fluorescent light when excited, where the light is detectable with a fluorescence detector. In some embodiments, the signal is or relates to light (*e.g*., visible light and/or ultraviolet light) that is detectable by a spectrophotometer. For example, light generated by a chemiluminescent reaction could be used as a signal. In some embodiments, the signal is or relates to radiation, *e.g.*, radiation emitted by radioisotopes, infrared radiation, *etc..* In certain embodiments, the signal is a direct or indirect indicator of a property of a physical entity. For example, a signal could be used as an indicator of amount and/or concentration of a nucleic acid in a biological sample and/or in a reaction vessel.

***"Specific":*** As used herein, the term "specific," when used in connection with an oligonucleotide primer, refers to an oligonucleotide or primer, under appropriate hybridization or washing conditions, is capable of hybridizing to the target of interest and not substantially hybridizing to nucleic acids which are not of interest. Higher levels of sequence identity are preferred and include at least 60%, 65%, 70%, 75%, 80%, 85%, 90%, 95%, 98%, 99%, or 100% sequence identity. In some embodiments, a specific oligonucleotide or primer contains at least 4, 6, 8, 10, 12, 14, 16, 18, 20, 22, 24, 26, 28, 30, 35, 40, 45, 50, 55, 60, 65, 70, or more bases of sequence identity with a portion of the nucleic acid to be hybridized or amplified when the oligonucleotide and the nucleic acid are aligned.

***"Stem-loop"*:** As used herein, the term "stem-loop", when used in connection with a nucleic acid structure, refers to a structure caused by an intramolecular base pairing typically occurring in single-stranded DNA or in RNA. The structure is also known as a hairpin or hairpin loop. Typically, it occurs when two regions of the same strand, usually complementary in nucleotide sequence when read in opposite directions, base-pair to form a double helix that ends in an unpaired loop, resulting in lollipop-shaped structure.

***"Substantially"*:** As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

***"Substantially complementary":*** As used herein, the term "substantially complementary" refers to two sequences that can hybridize under stringent hybridization conditions. The skilled artisan will understand that substantially complementary sequences need not hybridize along their entire length. In some embodiments, "stringent hybridization conditions" refer to hybridization conditions at least as stringent as the following: hybridization in 50% formamide, 5XSSC, 50 mM NaH₂PO₄, pH 6.8, 0.5% SDS, 0.1 mg/mL sonicated salmon sperm DNA, and 5XDenhart's solution at 42 °C overnight; washing with 2XSSC, 0.1% SDS at 45 °C; and washing with 0.2XSSC, 0.1% SDS at 45 °C. In some embodiments, stringent hybridization conditions should not allow for hybridization of two nucleic acids which differ over a stretch of 20 contiguous nucleotides by more than two bases.

### Detailed Description

The present invention provides, among other things, methods and compositions for multiplexed analysis of target nucleic acids (e.g., single or multiple targets simultaneously). As used herein, multiplexed analysis includes, but is not limited to, capturing, amplifying, detecting, analyzing and/or quantifying single target nucleic acid or multiple target nucleic acids simultaneously.

Various aspects of the invention are described in detail in the following sections. The use of sections is not meant to limit the invention.

### Target Nucleic Acids

Methods and compositions described herein may be used to analyze any target nucleic acids. In general, target nucleic acids may be any form of DNA, RNA, DNA/RNA chimera, or any combination thereof present in a sample.

### Samples

Any of a variety of samples may be suitable for use with methods disclosed herein including, but not limited to biological samples and chemical or recombinant preparations. Generally, any biological samples containing nucleic acids (*e.g.,* cells, tissue, *etc.*) may be used. Types of biological samples include, but are not limited to, cells, cell lysate, FFPE (FASP Protein Digestion) digests, tissues including tissue biopsies, whole blood, plasma, serum, urine, stool, saliva, cord blood, chorionic villus samples amniotic fluid, and transcervical lavage fluid. Cell cultures of any of the afore-mentioned biological samples may also be used in accordance with inventive methods, for example, chorionic villus cultures, amniotic fluid and/or amniocyte cultures, blood cell cultures (*e.g.,* lymphocyte cultures), *etc.* In some embodiments, biological samples comprise diseased cells such cancer or tumor cells. In some embodiments, biological samples are prenatal samples.

Thus, a typical biological sample suitable for the present invention contain heterogeneous nucleic acids. In some embodiments, a biological sample contains a mixture of nucleic acids from different cell types (e.g., normal cells and diseased cells such as tumor cells). In some embodiments, a biological sample (e.g., blood, serum or plasma) contains a mixture of maternal nucleic acids and fetal nucleic acids. Suitable samples may be unpurified or minimally purified biological samples or may be made of isolated nucleic acids DNA or RNA, urine, or plasma/serum.

In some embodiments, the present invention is used to analyze target nucleic acids that are present as rare events in a biological sample (also referred to as low abundance nucleic acid). In some embodiments, the amount of target nucleic acids detected by an inventive method of the present invention represents less than 1% (e.g., less than 0.5%, 0.1%, 0.01%, 0.001%, 0.0001%) of the total nucleic acids in a biological sample. In some embodiments, the amount of target nucleic acids detected by an inventive method of the present invention represents less than 1 out of a million of the total nucleic acids in a biological sample. In some embodiments, the amount of target nucleic acids detected by an inventive method of the present invention represents less than 1 out of 10 million of the total nucleic acids in a biological sample. In some embodiments, the present invention is used to analyze as few as one single copy of a nucleic acid target or up to one million or more copies of a nucleic acid target.

In some embodiments, suitable samples may be a chemical preparation or reaction mixture containing *in vitro* or recombinantly synthesized nucleic acids, such as, for example, siRNAs, mRNAs, microRNAs, aptamers, DNAs, plasmids, vectors, and the like.

### Different Targets

A target nucleic acid, in various embodiments, can be one that is found in a biological organism including, for example, a microorganism or infectious agent, or any naturally occurring, bioengineered or synthesized component thereof. In certain embodiments of the present invention, a target nucleic acid may be or contain a portion of a gene, a regulatory sequence, genomic DNA, cDNA, RNA including mRNA, rRNA, microRNA, small interfering RNA (siRNA), long noncoding RNA (lnc RNA), small nuclear RNA (snRNA), double stranded RNA (ds RNA) or any combination thereof. In certain embodiments of the present invention, a target nucleic acid may be a nucleic acid analogue or artificial nucleic acid, such as DNA/RNA chimeras.

In some embodiments, provided methods herein are used to detect and/or quantify miRNAs. miRNAs can be found in genomes of humans, animals, plants and viruses. According to the present invention, a target nucleic acid, in some embodiments, can be or comprise one or more miRNAs that is/are generated from endogenous hairpin-shaped transcripts. In some embodiments, a target nucleic acid can be or comprise one or more miRNAs that is/are transcribed as long primary transcripts (pri-microRNAs), for example, by RNA polymerase II enzyme in animals. There are a total of 1424 human miRNA genes currently listed in the miRNA database (available through the world wide web at microrna.sanger.ac.uk/sequences/ftp), which is equivalent to almost 3% of protein-coding genes. Many miRNAs are thought to be important in the regulation of gene expression. Typically, microRNAs are produced in precursor form and then processed to mature form by typically cleaving the 3' arm of the precursor stem-loop structure. Therefore, a precursor microRNA and a mature microRNA have identical 5' end but distinct 3' end. Selective end-labeling can be used to detect mature microRNA species without detection of precursor species by designing a capturing sequence complementary to the 3' end sequence.

### Capturing Target Nucleic Acids in a Sample

According to the present invention, target nucleic acids may be first captured by contacting a sample with one or more capturing probes. As used herein, the term "capturing probe" refers to a probe that comprises at least one target capturing sequence. As used herein, the term "target capturing sequence" refers to a nucleic acid sequence capable of binding to a target nucleic acid, e.g., microRNA. In some embodiments, a capturing probe comprises a single target capturing sequence and binds specifically to one distinct target nucleic acid. In some embodiments, a capturing probe comprises multiple (e.g., 2, 3, 4, 5, 10, or more) distinct target capturing sequences and binds to multiple (e.g., 2, 3, 4, 5, 10, or more) distinct target nucleic acids. Exemplary suitable target capturing sequences are described below.

In some embodiments, a capturing probe suitable for the present invention further includes one or more adapter binding sequences for binding adapters specifically designed to, e.g., serve as sites for polymerase chain reaction priming, reverse transcription, or modification by other DNA-modifying or RNA-modifying enzymes. Exemplary suitable adapters are described below.

According to the invention, the target capturing sequence (or sequences) and the adapter binding sequence (or sequences) are configured such that binding of both the one or more target nucleic acids and the one or more adapters to a capturing probe permits joining of the one or more adapters to the one or more target nucleic acids. For example, a capturing probe may include a target capturing sequence adjacent to an adapter binding sequence at 5' end, 3' end or both. In some embodiments, a capturing probe may include multiple target capturing sequences with each target capturing sequence framed by one or more adjacent adapter binding sequences. In some embodiments, adjacent means once both the target nucleic acid and the adapter are bound to the capturing probe, the 3' end of the target would abut the 5' end of the adapter or, alternatively, once both the target nucleic acid and the adapter bound to a capturing probe, the 5' end of the target would abut the 3' end of the adapter.

Suitable probes typically are of a length that is large enough to hybridize specifically with its target but not so large as to impede the hybridization process. The size may be dependent on the desired melting temperature of the target-probe complex or required specificity of target discrimination. In some embodiments, suitable probes contain about 10-200 nucleotides (e.g., 10-150, 10-100, 10-90, 10-80, 10-70, 10-60, 10-50, 10-40, 10-30, 10-20, 20-200, 20-150, 20-100, 20-90, 20-80, 20-70, 20-60, 20-50, 30-200, 30-150, 30-100, 30-90, 30-80, 30-70, 30-60, or 30-50 nucleotides). Various methods and softwares available in the art can be used to design specific probes.

Probes according to the invention may include natural nucleosides (*i.e.,* adenosine, thymidine, guanosine, cytidine, uridine, deoxyadenosine, deoxythymidine, deoxyguanosine, and deoxycytidine), nucleoside analogs (*e.g*., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g*., methylated bases), intercalated bases, modified sugars (*e.g*., 2'-fluororibose, ribose, 2'-deoxyribose, arabinose, and hexose), or modified phosphate groups (*e.g*., phosphorothioates and 5'-N-phosphoramidite linkages).

### Target capturing sequence

In some embodiments, a suitable target capturing sequence is specific to a target nucleic acid (e.g., DNA, mRNA, or microRNA). The term "specific" when used in connection with a hybridization probe refers to a sequence that can bind to its target under stringent conditions but not to other regions. In some embodiments, "stringent hybridization conditions" refer to hybridization conditions at least as stringent as the following: hybridization in 50% formamide, 5XSSC, 50 mM NaH₂PO₄, pH 6.8, 0.5% SDS, 0.1 mg/mL sonicated salmon sperm DNA, and 5XDenhart's solution at 42 °C overnight; washing with 2XSSC, 0.1% SDS at 45 °C; and washing with 0.2XSSC, 0.1% SDS at 45 °C. In some embodiments, stringent hybridization conditions should not allow for hybridization of two nucleic acids which differ over a stretch of 20 contiguous nucleotides by more than two bases. Other exemplary stringent conditions are well known in the art. Those skilled in the art understand how to estimate and adjust the stringency of hybridization conditions such that sequences having at least a desired level of complementarity will stably hybridize, while those having lower complementarity will not. For examples of hybridization conditions and parameters, see, e.g., Sambrook, et al., 1989, Molecular Cloning: A Laboratory Manual, Second Edition, Cold Spring Harbor Press, Plainview, N.Y.; Ausubel, F. M. et al. 1994, Current Protocols in Molecular Biology. John Wiley & Sons, Secaucus, N.J.

Thus, in some embodiments, a suitable target capturing sequence may contain a sequence substantially complementary to a target sequence, such as a microRNA. Typically, a target capturing sequence is based on a target-specific nucleotide sequence. In some embodiments, a target capturing sequence may contain a sequence substantially complementary to a sequence specific to an microRNA of interest, e.g., microRNAs indicative of certain cancer, diabetes, Alzheimer's or other diseases including but not limited to, let-7a, miR-21, miR-29b-2, miR-181b-1, miR-143, miR-145, miR-146a, miR-210, miR-221, miR-222, miR-10b, miR-15a, miR-16, miR-17, miR-18a, miR-19a, miR20a, miR-1, miR-29, miR-181, miR372, miR-373, miR-155, miR-101, miR-195, miR-29, miR-17-3p, miR-92a, miR-25, miR-223, miR-486, miR-223, mir-375, miR-99b, miR-127, miR-126, miR-184. In some embodiments, the capturing probes may contain one or more mismatch bases against target nucleic acid.

In some embodiments, a suitable capturing sequence may be designed to distinguish different variable species of target nucleic acids. For example, a capturing sequence can be designed to be complementary to a desired variable end nucleotide sequence. Only the binding of a desired target species will have a perfectly matching 3' end that abut the 5' end of the adapter sequence thereby permitting ligation of the adapter to the target. In particular embodiments, the present invention is used to distinguish a precursor-microRNA from a mature microRNA. Typically, a precursor-microRNA and mature microRNA have identical 5' region but distinct 3' region due to the cleavage of the 3' arm from the precursor form during the maturation process. In order to specifically detect a mature microRNA, a capturing sequence may be designed to be substantially complementary to the sequence at the 3' end of the mature microRNA. Therefore, only the binding of a correct mature microRNA to the capturing sequence would result in the perfectly matching 3' end of the microRNA abutting the 5' end of the adapter sequence permitting ligation of the adapter sequence to the target sequence.

In some embodiments, a capturing sequence for nucleic acid targets contains up to 50 nucleotides (e.g., up to 25, 20, 18, 16, 15, 14, 13, 12, 11, 10, 9, 8, 7, 6, 5, 4, 3, 2, or 1 nucleotides). In some embodiments, a capturing sequence is also chosen to ensure that the melting temperature (Tm) is between 10-50 °C in ligation buffer.

### Adapter binding sequence

Generally, an adapter binding sequence provides a binding site for an adapter, which typically is designed to, e.g., serve as sites for polymerase chain reaction priming, reverse transcription, or modification by other DNA-modifying or RNA-modifying enzymes. Suitable exemplary adapters are described below. Thus, an adapter binding sequence on a capturing probe is complementary or substantially complementary to an adapter. Typically, an adapter binding sequence and length are designed to such that (1) the melting temperature is between about 10-20 °C in ligation buffer, (2) the sequence is not significantly self-complementary in order to avoid formation of hairpin, other secondary structure or homodimer, and/or (3) complete DNA probes (with adapter and miRNA sequence) does not form appreciable hairpins or other secondary structures. In some embodiments, a suitable adapter sequence contains up to 20 nucleotides (e.g., up to 19, 18, 17, 16, 15, 14, 13, 12, 11, 10, 9, 8, 7, 6, 5, 4, 3, 2, or 1 nucleotides).

### Substrates

In some embodiments, capturing probes disclosed herein are associated with a substrate or object. For example, capturing probes may be attached or immobilized to a substrate or object, or embedded within the matrix of a substrate or object. Suitable substrates or objects may have a planer, spherical or non-spherical morphologies. Suitable substrates or objects may be solid, semi-solid, polymer, or the like. Exemplary suitable substrate may be made of a material selected from the group consisting of hydrogel, glass, photoresists, silica, polystyrene, polyethylene glycol, agarose, chitosan, alginate, PLGA, optical fiber, cellulose, and combination thereof. In some embodiments, suitable material is hydrogel. Suitable substrate may also be in various form, size and shape. For example, a suitable substrate may be a patterned planar substrate, microchips, plastics, beads, biofilms, or particles. In some embodiments, suitable substrate is a particle. For illustration purposes, particles are described in detail below.

Particles suitable for use in accordance with the present invention can be made of any material. Suitable particles can be biocompatible or non-biocompatible. Suitable particles can also be biodegradable or non-biodegradable.

In some embodiments, particles are hydrogels. In general, hydrogels comprise a substantially dilute crosslinked network. Water or other fluids can penetrate the network, forming such a hydrogel. In some embodiments, hydrogels suitable for use in the present invention are made of or comprise a hydrophilic polymer. For example, hydrophilic polymers may comprise anionic groups (*e.g*. phosphate group, sulphate group, carboxylate group); cationic groups (*e.g.* quaternary amine group); or polar groups (*e.g.* hydroxyl group, thiol group, amine group). In some embodiments, hydrogels are superabsorbent (*e.g*. they can contain over 99% water) and possess a degree of flexibility very similar to natural tissue, due to their significant water content. Both of weight and volume, hydrogels are fluid in composition and thus exhibit densities similar to those of their constituent liquids (e.g., water). The present invention encompasses the recognition that hydrogels are particularly useful in some embodiments of the present invention. In some embodiments, hydrogel is used to define aqueous compartments within a continuous hydrophobic phase that is immiscible or partially miscible with aqueous or hydrophilic solution. Without wishing to be bound to any particular theory, it is contemplated that hydrogels enable 1) ease of implementation with detection instruments, in particular, commercially available instruments without substantial modifications (e.g., flow cytometers), and 2) ease of incorporation of functional moieties (e.g., in a single lithography-polymerization step) without requiring surface functionalization.

Various additional materials and methods can be used to synthesize particles. In some embodiments, particles may be made of or comprise one or more polymers. Polymers used in particles may be natural polymers or unnatural (*e.g*. synthetic) polymers. In some embodiments, polymers can be linear or branched polymers. In some embodiments, polymers can be dendrimers. Polymers may be homopolymers or copolymers comprising two or more monomers. In terms of sequence, copolymers may be block copolymers, graft copolymers, random copolymers, blends, mixtures, and/or adducts of any of the foregoing and other polymers.

In some embodiments, particles of the present invention may be made of or comprise a natural polymer, such as a carbohydrate, protein, nucleic acid, lipid, *etc.* In some embodiments, natural polymers may be synthetically manufactured. Many natural polymers, such as collagen, hyaluronic acid (HA), and fibrin, which derived from various components of the mammalian extracellular matrix can be used in particles of the present invention. Collagen is one of the main proteins of the mammalian extracellular matrix, while HA is a polysaccharide that is found in nearly all animal tissues. Alginate and agarose are polysaccharides that are derived from marine algae sources. Some advantages of natural polymers include low toxicity and high biocompatibility.

Additional exemplary particle materials are described in International Patent Application WO2013166430.

In general, particles suitable for the present invention can be of any size. In some embodiments, suitable particles have a size greater than 1 µm up to about 1000 µm in at least one dimension (e.g., 1-500 µm, 1-450 µm, 1-400 µm, 1-350 µm, 1-300 µm, 1-250 µm, 1-200 µm, 1-150 µm, 1-100 µm, 1-50 µm, 2-50 µm, 2-100 µm, 50-1000 µm, 50-500 µm, 50-450 µm, 50-400 µm, 50-350 µm, 50-300 µm, 50-250 µm, 50-200 µm, 50-150 µm, 100-1000 µm, 100-500 µm, 100-450 µm, 100-400 µm, 100-350 µm, 100-300 µm, 100-250 µm, 100-200 µm, 100-150 µm in at least one dimension).

Suitable particles can have a variety of different shapes including, but not limited to, spheres, oblate spheroids, cylinders, ovals, ellipses, shells, cubes, cuboids, cones, pyramids, rods (*e.g*., cylinders or elongated structures having a square or rectangular cross-section), tetrapods (particles having four leg-like appendages), triangles, prisms, *etc.* In some embodiments, particles are rod-shaped. In some embodiments, particles are bar-shaped. In some embodiments, particles are bead-shaped. In some embodiments, particles are column-shaped. In some embodiments, particles are ribbon or chain-like. In some embodiments, particles can be of any geometry or symmetry. For example, planar, circular, rounded, tubular, ring-shaped, tetrahedral, hexagonal, octagonal particles, particles of other regular geometries, and/or particles of irregular geometries can also be used in the present invention. Additional suitable particles with various sizes and shapes are disclosed in US Patent No. 7,709,544 and US Patent No. 7,947,487 and can be used in the present invention.

Particles may have various aspect ratios of their dimensions, such as length/width, length/thickness, etc. Particles, in some embodiments, can have at least one dimension, such as length, that is longer than another dimension, such as width. According to the present invention, particles having at least one aspect ratio greater than one may be particularly useful in flow-through scanning (*e.g*., in a flow cytometer) to facilitate their self-alignment. In some embodiments, particles may have at least one aspect ratio of at least about 1.5:1, at least about 2:1, at least about 2.5:1, at least about 3:1, at least about 5:1, at least about 10:1, at least about 15:1, or even greater.

In some embodiments, capturing probes are attached to or embedded within one or more discrete regions of a particle. Such regions are referred to as probe regions. In some embodiments, each probe region bears anchors for attaching probes via, e.g., ligation-based approach. Ligation can be performed with three species (anchor, linker, and probe) or two species (hairpin anchor and probe). In some embodiments, probe region functionalization includes chemical modification, such as the use of peptide chemistry to attach aminated probes to carboxylated substrates using carbodiimide chemistry.

In some embodiments, a suitable particle comprises one or more coding regions (also referred to as encoding regions) bearing detectable moieties that give the identity of the probes attached to or embedded in the one or more probe regions of the same particle. Various detectable moieties may be used including fluorophores, chromophores, radioisotopes, quantum dots, nanoparticles and/or intercalating DNA/RNA dyes. Additional examples of detectable moieties are described in the Detectable Moieties section below.

In some embodiments, the one or more coding regions bear fluorophores such that the level of fluorescence is used for encoding. For example, fluorescence in each coding region can be distinguishable at multiple levels, e.g., up to 10 - 20 levels (e.g., up to 1, 2, 3, 4, 5, 6, 7, 8, 9, 10, 11, 12, 13, 14, 15, 16, 17, 18, 19, or 20 levels). As a non-limiting example, when three coding regions are used and 10 levels are distinguishable for each, it would allow up to 1000 (10x10x10) unique codes. Additionally or alternatively, multiple signals (e.g., different fluorescent colors) can be used for encoding. In some embodiments, each coding region has one signal distinct from each other. This may be accomplished by using blends of various fluorophores, with unique emission spectra.

In some embodiments, probe regions and coding regions are separated from one another by inert regions. In some embodiments, one or more probe-bearing regions and one or more coding regions overlap with each other. In some embodiments, a coding and probe-bearing region can be the same region.

### Capture Conditions

Capturing probes or substrates (e.g., particles) carrying capturing probes may be mixed with a sample under conditions that permit the capturing probes to capture one or more target nucleic acids in the sample. Various nucleic acid hybridization conditions and techniques may be employed as capture conditions. Typically, a stringent condition is used. In some embodiments, stringent hybridization conditions refer to hybridization conditions at least as stringent as the following: hybridization in 50% formamide, 5XSSC, 50 mM NaH2PO4, pH 6.8, 0.5% SDS, 0.1 mg/mL sonicated salmon sperm DNA, and 5XDenhart's solution at 42 °C overnight; washing with 2XSSC, 0.1% SDS at 45 °C; and washing with 0.2XSSC, 0.1% SDS at 45 °C. Other exemplary conditions are well known in the art. See, e.g., Sambrook, et al., 1989, Molecular Cloning: A Laboratory Manual, Second Edition, Cold Spring Harbor Press, Plainview, N.Y. Those skilled in the art understand how to estimate and adjust the stringency of hybridization conditions such that sequences having at least a desired level of complementarity will stably hybridize, while those having lower complementarity will not. For example, conditions may be tuned in order to give stringent capture by controlling: temperature, time, monovalent salt concentration, divalent salt concentration, dNTP concentration, or the addition of DMSO, formamide, polyethylene glycol, 2-pyrrolidone, or other agents that alter the kinetics of DNA duplex formation

### Coupling Adapters to Captured Target Nucleic acids

In some embodiments, in order to facilitate amplification, one or more adapters are coupled to the captured target nucleic acids prior to amplification. Typically, adapters include sequences specifically designed to serve as sites for polymerase chain reaction priming, reverse transcription, or modification by other DNA-modifying or RNA-modifying enzymes. In some embodiments, a suitable adapter contains a known universal oligonucleotide sequence so that the same adapter may be used to amplify different target nucleic acids. For example, a suitable adapter may contain forward or reverse primer recognition site for initiation of PCR. Same adapters may be coupled to different target nucleic acids and serve as PCR priming sites. Such adapters are also referred to as universal adapters. In some embodiments, a common universal adapter can be used to amplify multiple targets in a single reaction. Exemplary adapters design and sequences are described in the Examples section.

Adapters may be joined, linked, attached or coupled to the one or more targeted nucleic acids by enzymatic or chemical coupling. In some embodiments, a DNA or RNA ligase is used to link an adapter to a target nucleic acid. In some embodiments, a T4 DNA ligase is used to link an adapter to a target nucleic acid.

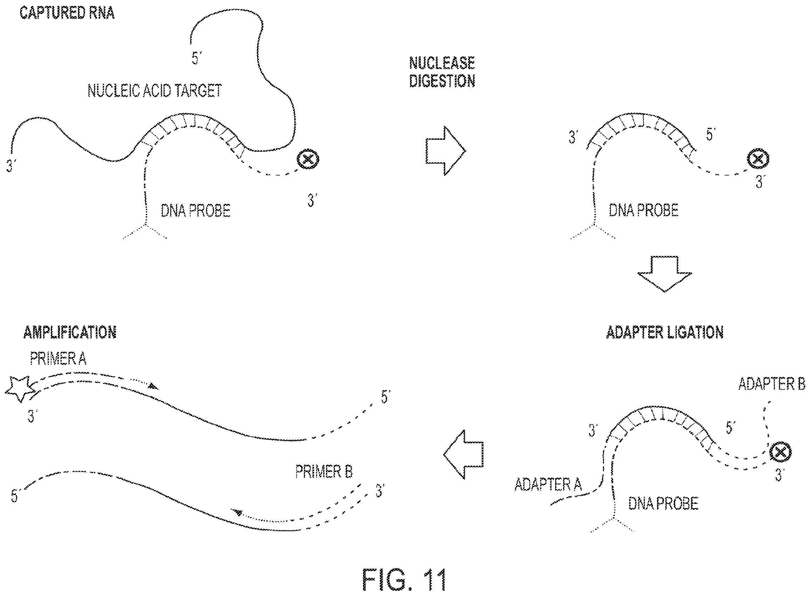

According to the invention, a suitable adapter contains a sequence complementary to an adapter binding sequence of a corresponding nucleic acid probe such that, once an adapter binds to the nucleic acid probe, the 5' or 3' end of the adapter abuts the 3' or 5' end of a target nucleic acid, respectively. In some embodiments, a captured target nucleic acid may have single-stranded 5' and/or 3' tail regions that are not bound to the capturing probe. In that case, the captured target is first treated by, e.g., nuclease or restriction enzyme digestion to remove single-stranded 5' and/or 3' regions to generate ligatable ends prior to the coupling of the one or more adapters. See Figure 11.

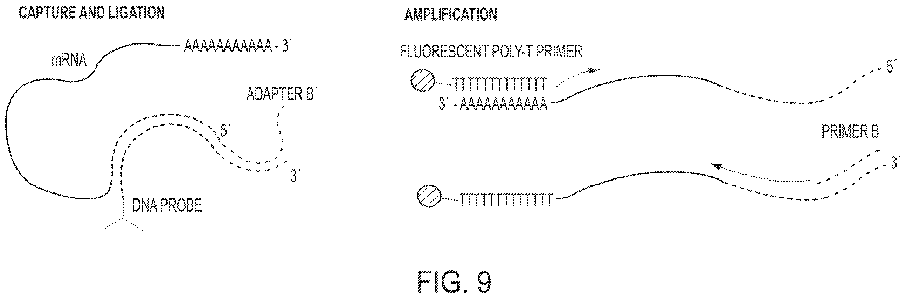

In some embodiments, a single adapter that is complementary to an adapter binding sequence of a corresponding nucleic acid probe is used. In some embodiments, a single adapter may be used together with a target specific primer sequence or a primer that bind to a sequence in the poly-A tail, e.g., a poly-T primer. See Figure 9.

Suitable lengths and sequences of an adaptor can be selected using methods well known and documented in the art. For example a suitable adapter may contain between 1 and 25 nucleotides in length (e.g., 1-20, 1-18, 1-16, 1-14, 1-12, 1-10, 5-20, 5-15, or 5-10 nucleotides).

Adapters may be DNA, RNA, or any type of nucleic acid analog including but not limited to DNA/RNA chimera. The nucleotides in adapters may be natural nucleosides (*i.e.,* adenosine, thymidine, guanosine, cytidine, uridine, deoxyadenosine, deoxythymidine, deoxyguanosine, and deoxycytidine), nucleoside analogs (*e.g*., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g*., methylated bases), intercalated bases, modified sugars (*e.g*., 2'-fluororibose, ribose, 2'-deoxyribose, arabinose, and hexose), or modified phosphate groups (*e.g*., phosphorothioates and 5'-N-phosphoramidite linkages).

In some embodiments, an adapter is not labeled. In some embodiments, an adapter is labeled to facilitate detection of amplified target nucleic acids. In some embodiments, a biotinylated adapter may be detected by a streptavidin reporter conjugated to a detectable moiety including, but not limited to, phycoerythrin, PE-Cy5, PE-Cy5.5, PE-Cy7, APC, PerCP, quantum dots, fluorophores or other detectable entities as described herein (see the "Detectable Entities" section below).

### Amplification of Target Nucleic Acids

According to the present invention, amplification refers to any methods known in the art for copying a target nucleic acid, thereby increasing the number of copies of a selected nucleic acid sequence. Amplification may be exponential or linear. A target nucleic acid may be either DNA or RNA. Typically, the sequences amplified in this manner form an "amplicon." Amplification may be accomplished with various methods including, but not limited to, polymerase chain reaction ("PCR"), transcription-based amplification, isothermal amplification, rolling circle amplification, etc.

Inventive methods described herein can give tunable degrees of amplification, as each PCR cycle will result in additional product molecules. A multiplex of specific capture reactions may be run in parallel with this method without increasing the complexity of the PCR amplification reaction, as a single or very few primer pairs (e.g., less than 5, 4, 3, or 2 pairs) may be used for each.

Amplification may be performed with relatively similar amount of each primer of a primer pair to generate a double stranded amplicon. However, asymmetric PCR or biased PCR may be used to amplify predominantly or exclusively a single stranded product as is well known in the art (e.g., Poddar et al. Molec. And Cell. Probes 14:25 -32, 2000). This can be achieved using each pair of primers by reducing the concentration of one primer significantly relative to the other primer of the pair (e.g., 2, 3, 4, 5, 10, 20, 30, 40, 50, or 100 fold difference). For example, PCR may be biased towards single-stranded amplified target nucleic acid through adding the forward primer at a concentration such that it is exhausted during the PCR reaction. In some embodiments, the ratio between the forward primer and the reverse primer may be less than 1:2, 1:3, 1:4, 1:5, 1:10, 1:20, 1:30, 1:40, 1:50, or 1:100. Additionally or alternatively, PCR may be biased towards single-stranded amplified target nucleic acid through designing a forward primer with a significantly lower annealing temperature than a reverse primer. For example, the annealing temperature of a forward primer may be about 2 °C, 3 °C, 4 °C, 5 °C, 6 °C, 7 °C, 8 °C, 9 °C, or 10 °C lower than the annealing temperature of a reverse primer. Amplification by asymmetric or biased PCR is generally linear. A skilled artisan will understand that different amplification methods may be used together.

In some embodiments, bridge PCR amplification can be used for amplifying the captured target nucleic acid. Typically, bridge PCR involves universal amplification reaction, whereby a nucleic acid is treated such that the ends of the different targets all contain the same DNA sequence (e.g., universal adapter). Targets with universal ends can then be amplified in a single reaction with a single pair of amplification primers. Targets will then be separated into a single molecule level prior to amplification to ensure that the amplified molecules form discrete populations that can then be further analyzed. Such separations can be performed either in emulsions, on a surface, or within a gel.

Various exemplary methods of bridge amplification are known in the art and can be modified to practice the present invention. For example, various bridge amplification methods are described in U.S. Patent No. 7,115,400, U.S. Publication No. 20090226975, and Bing D. H. et al. "Bridge Amplification: A Solid Phase PCR System for the Amplification and Detection of Allelic Differences in Single Copy Genes," Seventh International Symposium on Human Identification (available through the world wide web at promega.com/geneticidproc/ussymp7proc/0726).

In some embodiments, rolling circle PCR (isothermal PCR) is used to amplify nucleic acids. Guidance for selecting conditions and reagents for RCR reactions is available in many references available to those of ordinary skill, as evidence by the following: Kool, U.S. Pat. No. 5,426,180; Lizardi, U.S. Pat. Nos. 5,854,033 and 6,143,495; Landegren, U.S. Pat. No. 5,871,921; and the like. Generally, rolling circle PCR reaction components comprise single stranded DNA circles, one or more primers that anneal to DNA circles, a DNA polymerase having strand displacement activity to extend the 3' ends of primers annealed to DNA circles, nucleoside triphosphates, and a conventional polymerase reaction buffer. Such components are combined under conditions that permit primers to anneal to DNA circles and be extended by the DNA polymerase to form concatemers of DNA circle complements. An exemplary rolling circle PCR reaction protocol is as follows: In a 50 µL reaction mixture, the following ingredients are assembled: 2-50 pmol circular DNA, 0.5 units/µL phage ϕ29 DNA polymerase, 0.2 µg/µL BSA, 3 mM dNTP, 1× ϕ29 DNA polymerase reaction buffer (Amersham). The rolling circle PCR reaction is carried out at 30 °C for 12 hours. In some embodiments, the concentration of circular DNA in the polymerase reaction may be selected to be low (approximately 10-100 billion circles per ml, or 10-100 circles per picoliter) to avoid entanglement and other intermolecular interactions.

Various enzymes may be used to facilitate amplification. In some embodiments, polymerases that include reverse transcription activity, such as Tth polymerase and Pyrophage 3713 polymerase may be used so that one enzyme can generate cDNA and PCR amplify in the same reaction. In some embodiments, the reverse transcription and/or PCR reaction can occur either in the presence of the substrate (e.g., hydrogel particles). In some embodiments, captured target nucleic acids are first separated from the substrate (e.g., hydrogel particles).

Typically, amplification may be carried out in a reaction mixture containing enzymes, dNTPs, primers, labeling agents (e.g., detectable entities) and other buffering reagents.

### Enzymes

In some embodiments, amplification of target nucleic acids, in particular, target RNAs, employs a single enzyme that has both DNA polymerase activity and reverse transcriptase activity (referred to as a one-enzyme system in some cases). Examples of such enzymes include, but are not limited to, Pyrophage and TtH.

In some embodiments, reverse transcription is catalyzed by one enzyme and PCR amplification is carried out by a second enzyme (referred to as a two-enzyme system in some cases). As non-limiting examples, polymerases such as Taq, Bst, or Phi29 may be used.

Other examples of nucleic acid polymerases that can be used in the present invention are DNA polymerase (Klenow fragment, T4 DNA polymerase), heat-stable DNA polymerases from a variety of thermostable bacteria (such as Taq, VENT, Pfu, Tfl DNA polymerases) as well as their genetically modified derivatives (TaqGold, VENTexo, Pfu exo).

For rolling circle amplification, a target nucleic acid is typically circularized first using, for example, a DNA/RNA ligase. Thus, in some embodiments, a ligase may be included in an amplification reaction mixture.

### Primers

According to the present invention, primer refers to a short single-stranded oligonucleotide capable of hybridizing to a complementary sequence in a nucleic acid sample or adapter. Typically, a primer serves as an initiation point for template dependent DNA synthesis. Deoxyribonucleotides can be added to a primer by a DNA polymerase. In some embodiments, such deoxyribonucleotides addition to a primer is also known as primer extension. The term primer, as used herein, includes all forms of primers that may be synthesized including peptide nucleic acid primers, locked nucleic acid primers, phosphorothioate modified primers, labeled primers, and the like. A "primer pair" or "primer set" for a PCR reaction typically refers to a set of primers typically including a "forward primer" and a "reverse primer." As used herein, a "forward primer" refers to a primer that anneals to the anti-sense strand of dsDNA. A "reverse primer" anneals to the sense-strand of dsDNA.

Depending on the nature of PCR, a single primer or a pair of primers may be used. For example, a single primer may be used in rolling circle amplification. A pairs of primers are typically used for other forms of PCR (e.g., forward and reverse primers). As discussed above, the ratio between the forward and reverse primers may be adjusted for asymmetric or biased PCR.

In some embodiments, primers may be present in a solution-phase PCR reaction, but also one or both of the primers may be attached to the solid support at the 5'-end. This would allow for a bridge PCR or bridge PCR-like reaction to be carried out. In some embodiments, microparticles used to capture target nucleic acids may be composed of a tunable hydrogel matrix composed of mostly aqueous phase. When such particle hydrogels, composed mostly of water, are dispersed in an hydrophobic solution, their contents are prevented from escaping in the solution medium and can be considered discrete aqueous reactors. In some such embodiments, particles are large enough to allow for sufficient reagent volume to be defined directly by the hydrogel particle dimensions. As non-limiting examples, a suitable particle may be from 2-50 microns in diameter, or from 50-200 microns in diameter. Other suitable sizes are described throughout the specification.

### Labeling amplified target nucleic acids

According to the present invention, there are multiple ways to produce labeled amplified target nucleic acids. In some embodiments, amplified nucleic acids are labeled as a result of using a labeled reverse primer for amplification. In some embodiments, amplified nucleic acids are labeled as a result of using labeled dNTPs during amplification. In some embodiments, amplified nucleic acids are labeled as a result of using intercalating dyes during amplification.

### Detectable entities

Any of a wide variety of detectable agents can be used in the practice of the present invention. Suitable detectable entities include, but are not limited to: various ligands, radionuclides; fluorescent dyes; chemiluminescent agents (such as, for example, acridinum esters, stabilized dioxetanes, and the like); bioluminescent agents; spectrally resolvable inorganic fluorescent semiconductors nanocrystals (i.e., quantum dots); metal nanoparticles (e.g., gold, silver, copper, platinum, etc.); nanoclusters; paramagnetic metal ions; enzymes; colorimetric labels (such as, for example, dyes, colloidal gold, and the like); biotin; dioxigenin; haptens; and proteins for which antisera or monoclonal antibodies are available.

In some embodiments, the detectable moiety is biotin. Biotin can be bound to avidins (such as streptavidin), which are typically conjugated (directly or indirectly) to other moieties (e.g., fluorescent moieties) that are detectable themselves.

Below are described some non-limiting examples of other detectable moieties.

### Fluorescent dyes

In certain embodiments, a detectable moiety is a fluorescent dye. Numerous known fluorescent dyes of a wide variety of chemical structures and physical characteristics are suitable for use in the practice of the present invention. A fluorescent detectable moiety can be stimulated by a laser with the emitted light captured by a detector. The detector can be a charge-coupled device (CCD) or a confocal microscope, which records its intensity.

Suitable fluorescent dyes include, but are not limited to, fluorescein and fluorescein dyes (e.g., fluorescein isothiocyanine or FITC, naphthofluorescein, 4',5'-dichloro-2',7'-dimethoxyfluorescein, 6-carboxyfluorescein or FAM, etc.), carbocyanine, merocyanine, styryl dyes, oxonol dyes, phycoerythrin, erythrosin, eosin, rhodamine dyes (e.g., carboxytetramethyl-rhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (e.g., methoxycoumarin, dialkylaminocoumarin, hydroxycoumarin, aminomethylcoumarin (AMCA), etc.), Oregon Green Dyes (e.g., Oregon Green 488, Oregon Green 500, Oregon Green 514., etc.), Texas Red, Texas Red-X, SPECTRUM RED™, SPECTRUM GREEN™, cyanine dyes (e.g., CY-3™, CY-5™, CY-3.5™, CY-5.5™, etc.), ALEXA FLUOR™ dyes (e.g., ALEXA FLUOR™ 350, ALEXA FLUOR™ 488, ALEXA FLUOR™ 532, ALEXA FLUOR™ 546, ALEXA FLUOR™ 568, ALEXA FLUOR™ 594, ALEXA FLUOR™ 633, ALEXA FLUOR™ 660, ALEXA FLUOR™ 680, etc.), BODIPY™ dyes (e.g., BODIPY™ FL, BODIPY™ R6G, BODIPY™ TMR, BODIPY™ TR, BODIPY™ 530/550, BODIPY™ 558/568, BODIPY™ 564/570, BODIPY™ 576/589, BODIPY™ 581/591, BODIPY™ 630/650, BODIPY™ 650/665, etc.), IRDyes (e.g., IRD40, IRD 700, IRD 800, etc.), and the like. For more examples of suitable fluorescent dyes and methods for coupling fluorescent dyes to other chemical entities such as proteins and peptides, see, for example, "The Handbook of Fluorescent Probes and Research Products", 9th Ed., Molecular Probes, Inc., Eugene, OR. Favorable properties of fluorescent labeling agents include high molar absorption coefficient, high fluorescence quantum yield, and photostability. In some embodiments, labeling fluorophores exhibit absorption and emission wavelengths in the visible (i.e., between 400 and 750 nm) rather than in the ultraviolet range of the spectrum (i.e., lower than 400 nm).

A detectable moiety may include more than one chemical entity such as in fluorescent resonance energy transfer (FRET). Resonance transfer results an overall enhancement of the emission intensity. For instance, see Ju et. al. (1995) Proc. Nat'l Acad. Sci. (USA) 92: 4347. A suitable detectable moiety can be an intercalating DNA/RNA dye that have dramatic fluorescent enhancement upon binding to double-stranded DNA/RNA. Examples of suitable dyes include, but are not limited to, SYBR™ and Pico Green (from Molecular Probes, Inc. of Eugene, Oreg.), ethidium bromide, propidium iodide, chromomycin, acridine orange, Hoechst 33258, Toto-1, Yoyo-1, and DAPI (4',6-diamidino-2-phenylindole hydrochloride). Additional discussion regarding the use of intercalation dyes is provided by Zhu et al., Anal. Chem. 66:1941-1948 (1994).

In certain embodiments, a detectable moiety is an enzyme. Examples of suitable enzymes include, but are not limited to, those used in an ELISA, e.g., horseradish peroxidase, beta-galactosidase, luciferase, alkaline phosphatase, etc. Other examples include beta-glucuronidase, beta-D-glucosidase, urease, glucose oxidase, etc. An enzyme may be conjugated to a molecule using a linker group such as a carbodiimide, a diisocyanate, a glutaraldehyde, and the like.

### Radioactive isotopes

In certain embodiments, a detectable moiety is a radioactive isotope. For example, a molecule may be isotopically-labeled (i.e., may contain one or more atoms that have been replaced by an atom having an atomic mass or mass number different from the atomic mass or mass number usually found in nature) or an isotope may be attached to the molecule. Non-limiting examples of isotopes that can be incorporated into molecules include isotopes of hydrogen, carbon, fluorine, phosphorous, copper, gallium, yttrium, technetium, indium, iodine, rhenium, thallium, bismuth, astatine, samarium, and lutetium (i.e., ³H, ¹³C, ¹⁴C, ¹⁸F, ¹⁹F, ³²P, ³⁵S, ⁶⁴Cu, ⁶⁷Cu, ⁶⁷Ga, ⁹⁰Y, ⁹⁹mTc, ¹¹¹In, ¹²⁵I, ¹²³I, ¹²⁹I, ¹³¹I, ¹³⁵I, ¹⁸⁶Re, ¹⁸⁷Re, ²⁰¹Tl, ²¹²Bi, ²¹³Bi, ²¹¹At, ¹⁵³Sm, ¹⁷⁷Lu).

In some embodiments, signal amplification is achieved using labeled dendrimers as the detectable moiety (see, e.g., Physiol Genomics 3:93-99, 2000). Fluorescently labeled dendrimers are available from Genisphere (Montvale, N.J.). These may be chemically conjugated to the oligonucleotide primers by methods known in the art.

### Recapturing Amplified Labeled Target Nucleic Acids

In some embodiments, amplification product may then be incubated with re-capturing probes such that the amplified one or more target nucleic acids can be detected and/or analyzed. In general, re-capturing probes may be designed similarly to capturing probes to specifically bind to amplified target nucleic acids. In some embodiments, each of the re-capturing probes contains single target capturing sequence and binds specifically to one distinct target nucleic acid. In some embodiments, each of the re-capturing probes contains multiple distinct target capturing sequences and can bind multiple distinct target nucleic acids. In some embodiments, re-capturing probes are identical to capturing probes. In some embodiments, capturing probes may be used to re-capture amplified target nucleic acids. Like in the capturing probes, the targeting capturing sequence of the re-capturing probes is substantially complementary to the target nucleic acid. In some embodiments, the target capturing sequence of the re-capturing probes may contain one or more mismatch bases against target nucleic acid.

The size of a re-capturing probe may be dependent on the desired melting temperature of the target-probe complex or required specificity of target discrimination. In some embodiments, suitable probes contains about 10-70 nucleotides (e.g., 10-60, 10-50, 10-40, 10-30, 10-25, 10-20, 15-70, 15-60, 15-50, 15-40, 15-30, 15-25, 20-70, 20-60, 20-50, 20-40, 20-30 nucleotides). Various methods and softwares available in the art can be used to design specific probes.

Nucleic acid probes may include natural nucleosides (*i.e.,* adenosine, thymidine, guanosine, cytidine, uridine, deoxyadenosine, deoxythymidine, deoxyguanosine, and deoxycytidine), nucleoside analogs (*e.g*., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g*., methylated bases), intercalated bases, modified sugars (*e.g*., 2'-fluororibose, ribose, 2'-deoxyribose, arabinose, and hexose), or modified phosphate groups (*e.g*., phosphorothioates and 5'-N-phosphoramidite linkages).

Re-capturing probes may be attached to or embedded in various substrates (e.g., hydrogel particles) described herein. In some embodiments, the original target capture particles can be used to bind the resulting amplicon, as the sequence will be identical to the original adapted nucleic acid target. This method can give tunable degrees of amplification, as each PCR cycle will result in additional product molecules. A multiplex of specific capture reactions may be run in parallel with this method without increasing the complexity of the PCR amplification reaction, as a single or very few primer pairs (e.g., less than 5, 4, 3, or 2 pairs) will be used for each.

Typically, stringent conditions are used to re-capture amplified target nucleic acids following amplification. Various stringent conditions are described throughout the specification and are well known in the art. Those skilled in the art understand how to estimate and adjust the stringency of hybridization conditions such that sequences having at least a desired level of complementary will stably hybridize, while those having lower complementary will not. For examples of hybridization conditions and parameters, see, e.g., Sambrook, et al., 1989, Molecular Cloning: A Laboratory Manual, Second Edition, Cold Spring Harbor Press, Plainview, N.Y.; Ausubel, F. M. et al. 1994, Current Protocols in Molecular Biology. John Wiley & Sons, Secaucus, N.J. In some embodiments, re-capturing conditions are substantially more stringent than initial conditions for capturing target nuclei acids in a sample.

### Detection and Quantification

Various methods can be used to detect, quantify and/or analyze target nucleic acids. Typically, target nucleic acids may be detected through detecting signal generated by detectable entity associated with the re-captured amplified target nucleic acids. In some embodiments, signals emanate from an entity (*e.g.,* a detectable moiety) that is physically associated with a probe at the time the signal is detected. In some embodiments, signals emanate from an entity that is not physically associated with a probe at the time the signal is detected. In some embodiments, the amount of target nucleic acids may be determined by quantifying the amount of signals detected relative to a reference or control.

In some embodiments, detectable signals are optical signals, such as, for example, fluorescent or luminescent signals. Various devices may be used to detect a signal associated with a target nucleic acid. Typically the signal is an optical signal and an optical detector is used. Optical detectors can include one or more of photodiodes (*e.g*., avalanche photodiodes), a fiber-optic light guide leading, for example, to a photomultiplier tube, a microscope, and/or a video camera (*e.g*., a charged couple device (CCD) camera), or a flow-through device such as a flow cytometer.

Exemplary methods and apparatus for characterization and quantification of multifunctional objects are discussed in International Patent Application WO2013134633 and U.S. Patent Application Publication No. 2013/0244909.

In certain embodiments, signals are converted to numerical values using standard software known in the art. In some embodiments, signals (or numerical values representative of signals) are normalized based on background signals. Any of a variety of software programs known in the art may be used to analyze signals as described herein, including, but not limited to, GENEPIX PRO™ 4.0.1.12 software (Axon Instruments, USA), Feature Extraction (Agilent), Matlab (Mathworks), and the like. Exemplary software program for converting and quantifying signals detected by flow-cytometer from a multifunctional particle as described herein are described in International application WO2013134633.

### Applications

The present invention may be used for various applications. For example, the present invention may be used for diagnosis and prognosis of diseases, disorders or conditions based on detection or quantification of a target nucleic acid (e.g., microRNA, DNA or mRNA) in a biological sample. In some embodiments, captured target nucleic acids may also be used for generating a nucleic acid library and/or sequencing. Exemplary applications of the present invention are described in detail below and in the Examples section.

### Diagnosis

In some embodiments, the present invention can be used to diagnose or prognose a variety of diseases including, but not limited to, cancer (e.g., lung cancer, breast cancer, stomach cancer, pancreatic cancer, lymphoma, leukemia, colon cancer, liver cancer, etc.), diabetes, neurodegenerative diseases (e.g., Alzheimer's), infectious diseases, and genetic diseases.

Representative bacterial infectious agents which can be detected and/or determined by the present invention include, but are not limited to, Escherichia coli, Salmonella, Shigella, Klebsiella, Pseudomonas, Listeria monocytogenes, Mycobacterium tuberculosis, Mycobacterium aviumintracellulare, Yersinia, Francisella, Pasteurella, Brucella, Clostridia, Bordetella pertussis, Bacteroides, Staphylococcus aureus, Streptococcus pneumonia , B-Hemolytic strep., Corynebacteria, Legionella, Mycoplasma, Ureaplasma, Chlamydia, Neisseria gonorrhea, Neisseria meningitides, Hemophilus influenza, Enterococcus faecalis, Proteus vulgaris, Proteus mirabilis, Helicobacter pylori, Treponema palladium, Borrelia burgdorferi, Borrelia recurrentis, Rickettsial pathogens, Nocardia , and Acitnomycetes.

Representative fungal infectious agents which can be detected and/or determined by the present invention include, but are not limited to, Cryptococcus neoformans, Blastomyces dermatitidis, Histoplasma capsulatum, Coccidioides immitis, Paracoccidioides brasiliensis, Candida albicans, Aspergillus fumigautus, Phycomycetes (Rhizopus), Sporothrix schenckii, Chromomycosis, and Maduromycosis.

Representative viral infectious agents which can be detected and/or determined by the present invention include, but are not limited to, human immunodeficiency virus, human T-cell lymphocytotrophic virus, hepatitis viruses (e.g., Hepatitis B Virus and Hepatitis C Virus), Epstein-Barr Virus, cytomegalovirus, human papillomaviruses, orthomyxo viruses, paramyxo viruses, adenoviruses, corona viruses, rhabdo viruses, polio viruses, toga viruses, bunya viruses, arena viruses, rubella viruses, and reo viruses.

Representative parasitic agents which can be detected and/or determined by the present invention include, but are not limited to, Plasmodium falciparum, Plasmodium malaria, Plasmodium vivax, Plasmodium ovale, Onchoverva volvulus, Leishmania, Trypanosoma spp., Schistosoma spp., Entamoeba histolytica, Cryptosporidum, Giardia spp., Trichimonas spp., Balatidium coli, Wuchereria bancrofti, Toxoplasma spp., Enterobius vermicularis, Ascaris lumbricoides, Trichuris trichiura, Dracunculus medinesis, trematodes, Diphyllobothrium latum, Taenia spp., Pneumocystis carinii, and Necator americanis.

The present invention can also be useful for detection and/or determination of drug resistance by infectious agents. For example, vancomycin-resistant Enterococcus faecium, methicillin-resistant Staphylococcus aureus, penicillin-resistant Streptococcus pneumoniae, multi-drug resistant Mycobacterium tuberculosis, and AZT-resistant human immunodeficiency virus can be identified with the present invention.

Genetic diseases can also be detected and/or determined by the process of the present invention. This can be carried out by prenatal or post-natal screening for chromosomal and genetic aberrations or for genetic diseases. Examples of detectable genetic diseases include, but are not limited to: 21 hydroxylase deficiency, cystic fibrosis, Fragile X Syndrome, Turner Syndrome, Duchenne Muscular Dystrophy, Down Syndrome or other trisomies, heart disease, single gene diseases, HLA typing, phenylketonuria, sickle cell anemia, Tay-Sachs Disease, thalassemia, Klinefelter Syndrome, Huntington Disease, autoimmune diseases, lipidosis, obesity defects, hemophilia, inborn errors of metabolism, and diabetes.

Cancers which can be detected and/or determined by the process of the present invention generally involve oncogenes, tumor suppressor genes, or genes involved in DNA amplification, replication, recombination, or repair. Examples of these include, but are not limited to: BRCA1 gene, p53 gene, APC gene, Her2/Neu amplification, Bcr/Ab1, K-ras gene, and human papillomavirus Types 16 and 18. Various aspects of the present invention can be used to identify amplifications, large deletions as well as point mutations and small deletions/insertions of the above genes in the following common human cancers: leukemia, colon cancer, breast cancer, lung cancer, prostate cancer, brain tumors, central nervous system tumors, bladder tumors, melanomas, liver cancer, osteosarcoma and other bone cancers, testicular and ovarian carcinomas, head and neck tumors, and cervical neoplasms.

In the area of environmental monitoring, the present invention can be used, for example, for detection, identification, and monitoring of pathogenic and indigenous microorganisms in natural and engineered ecosystems and microcosms such as in municipal waste water purification systems and water reservoirs or in polluted areas undergoing bioremediation. It is also possible to detect plasmids containing genes that can metabolize xenobiotics, to monitor specific target microorganisms in population dynamic studies, or either to detect, identify, or monitor genetically modified microorganisms in the environment and in industrial plants.

The present invention can also be used in a variety of forensic areas, including, for example, for human identification for military personnel and criminal investigation, paternity testing and family relation analysis, HLA compatibility typing, and screening blood, sperm, or transplantation organs for contamination.

In the food and feed industry, the present invention has a wide variety of applications. For example, it can be used for identification and characterization of production organisms such as yeast for production of beer, wine, cheese, yoghurt, bread, etc. Another area of use is with regard to quality control and certification of products and processes (e.g., livestock, pasteurization, and meat processing) for contaminants. Other uses include the characterization of plants, bulbs, and seeds for breeding purposes, identification of the presence of plant-specific pathogens, and detection and identification of veterinary infections.

### Sequencing

Library construction and sample preparation remain two major hurdles in the widespread clinical adoption of sequencing. The present invention can be adapted in order to generate libraries of controlled size, each bearing the desired platform-specific PCR adapters and/or sequence barcodes for sample pooling. This method is appropriate for generating libraries from either fragmented DNA or directly from RNA.

In one embodiment, a multiplex mixture of solid or semi-solid supports is mixed directly with the biological sample of interest. The solid or semi-solid supports bearing oligonucleotide probes, with random sequences or target specific sequences with adapter-specific regions on the 3' and 5' end of the capture probe, are mixed with the biological samples of interest. Post-capture, a sticky-end or blunt-end ligation step will be performed with a specific ligase enzyme. This is followed by a limited PCR or PCR-like reaction, which amplifies the bound molecules but retains the relative abundance of the target species.

For preparations of RNA, a polymerase with reverse transcription activity, or a reverse transcriptase must be used, in order to convert the bound RNA-DNA hybrid into a DNA amplicon product. Following the limited PCR cycling, the amplified product can be collected, quantified, and used directly as input for sequencing sample preparation. This includes reactions such as emulsion PCR (Ion Torrent PGM™, Ion Torrent Proton™, Roche 454™) or bridge PCR (Illumina™). By altering primer design and adapter design, libraries can be modified to work on any of these sequencing platforms, or to allow sample multiplexing through specific barcode sequence inclusion in the nucleic acid adapters. This method may provide size selection through hydrogel pore size and capture oligonucleotide design, as well as allowing direct library preparation from RNA, or from samples with limited starting material without a specific reverse transcription reaction. Additionally, due to the bioinert or nonfouling properties of some hydrogel substrates (Polyethylene glycol, alginate, chitosan, polylactic/glycolic acid), these substrates can be used to capture nucleic acids from unpurified or minimally purified biological samples.

### EXAMPLES

### Example 1: microRNA Capture and Signal Amplification

This example demonstrates that exemplary particles can capture microRNA and that their signal can be amplified via PCR or a PCR-like reaction and the creation of a fluorescent product. Exemplary methods are described in detail below.

MicroRNAs are an emerging class of small RNA biomarkers that have been shown to regulate the majority of genes in eukaryotic organisms. These RNA molecules have also been shown to be highly stable in blood. Several emerging technologies have sought to accurately detect and quantify microRNAs in a multiplexed format, but many technologies suffer from poor sensitivity, low multiplexing, or low sample throughput. The method outlined above is well-suited to the ultra-sensitive detection of these and other small-RNA markers. In this embodiment, the solid or semisolid support will comprise a mixture of one or more types of hydrogel particles each bearing a unique encoding and capture probe. The microRNA target will be captured and adapted via a ligation reaction as described above. This will result in a DNA-RNA chimeric molecule. This molecule can be amplified via a PCR or PCR-like reaction, by utilizing an enzyme with reverse transcription activity. By using a fluorescent reverse primer and biasing the reaction conditions towards single-stranded product, it is possible to generate significant amounts of ssDNA product with identical sequence to the initial adapted microRNA-DNA chimeric target.

In order to selectively bias the reaction towards fluorescent single-stranded DNA product, a high ratio of reverse fluorescent primer to forward non-fluorescent primer may be used. When the forward primer is completely consumed, the reaction will continue generating product with only the fluorescent reverse primer. Another method of producing single-stranded product is by designing a forward primer with a lower melting temperature than the fluorescent reverse primer. The PCR cycling conditions can be set such that cycles begin symmetrically with both primers hybridizing and forming double-stranded DNA product. After a set number of cycles, the annealing temperature should be increased, leading to extension of only the fluorescent reverse primer, and biasing the reaction towards fluorescent single-stranded product. This product will be specifically captured on the semi-solid hydrogel particle support during the PCR reaction, or during a final incubation with controlled hybridization conditions. Limited PCR cycles have been shown to give 100-1000x amplification of each bound microRNA target. This reaction can be multiplexed from 1 to 1000+ microRNA targets with a single primer pair. This results in minimal primer-primer or primer-probe interaction, minimizing off-target product formation.

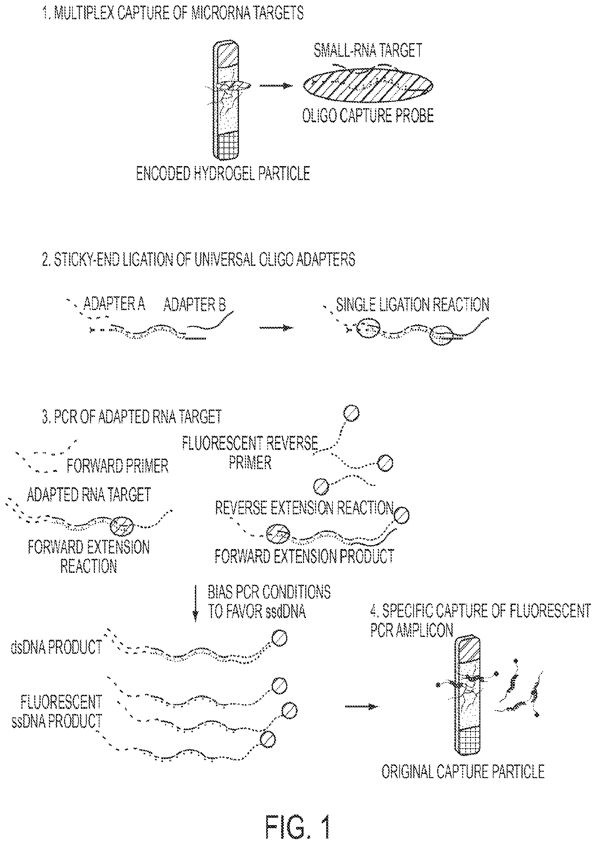

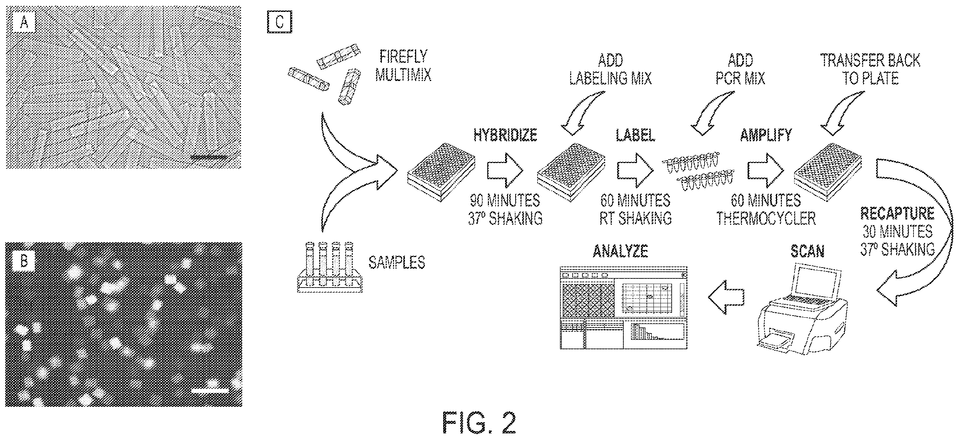

An example of this multiplexed microRNA detection method is illustrated in Figure 1 and Figure 2. This method would be perfectly amenable to other small-RNAs, including piwi-interacting-RNAs (piRNAs), siRNAs, and rasiRNAs.

### Probes

Hydrogel particles are produced via stop-flow lithography, developed by Pregibon, Doyle, et al., such that rod-like particles are made, each containing a unique DNA probe and fluorescent code. Each DNA probe consists of a region that is complementary to a specific small RNA, and flanking regions on both the 5' and 3' ends that are common to all probes.

### Hybridization

In preparation for running the Firefly Plasma/Serum (PS) protocol, it is important that all work surfaces be prepared in a manner reflective of best practices to minimize PCR contamination. These include, but may not be limited to, bleaching the work surface and then exposing it to UV for a minute, or cleaning down the work surface with a DNA-removing cleaner like LookOut DNA Erase (Sigma). Filter tips should be used at all time.

The first step in the assay is to fully resuspend the particle mastermix with repeated inverting and vortexing. 35 µl of particles is added to each experimental well of a filter plate (Millipore, MSBVN1210) with repeated mixing between aliquotting to ensure the mix remains fully suspended. Vacuum pressure is then applied to the filter plate to remove any liquid, leaving the particles behind in the wells. 25 µl of Hybridization Buffer, formulated to promote specific hybridization, is added to each well, followed by 25 µl of the miRNA-containing sample to be quantified. This mixture is incubated for 90 minutes at 37 °C to allow complementary miRNAs to fully bind to particle-bound probes. After hybridization, the particles are washed twice with 200 µl of a Rinse Solution to remove non-complementary RNA and partially complementary miRNAs that have not formed stable duplexes with probe molecules.

### Labeling

After hybridization, the particles are resuspended in 75 µl of ligation solution, and shaken at room temperature for 1 hour. The ligation solution consists of Tris-EDTA, T4 RNA Ligase 2, ATP, MgCl2, Tween-20, glycerol, a 5' adapter and a 3' adapter. 5' adapter sequences include: rGrCrUrArGrUrCrCrUrArUrGrCrArArUrGrUrCrArUrArArArUrArUrArArArU (SEQ ID NO: 1), CCTATGCAATGTCArUrArArArUrArUrArArArU (SEQ ID NO: 2), GGCTGAGTGCAGTGCGAGrArArArUrArUrArArArU (SEQ ID NO: 3) and GGTTGGCCACGTGACTTGATCTTrArArArUrArUrArArArU (SEQ ID NO: 4). In SEQ ID NO: 1-4, "r" before G, C, A or U denotes a ribonucleotide. 3' adapter sequences include: /5Phos/TAATAAAATATATCCGTCGATAAGCGGATCTATC (SEQ ID NO: 5), /5Phos/TAATAAAATATATCCGTCGATAAGCG (SEQ ID NO: 6), /5Phos/TTTAAAATATATCCGTCGATAAGCG (SEQ ID NO: 7), /5Phos/TAATAAAATATATCCGTCGATAAGCG (SEQ ID NO: 8), and /5Phos/TTTAAAATATATCAAGCGTCAATTAGCGCGA (SEQ ID NO: 9).

Subsequently the particles are washed twice with 200 µl of a stringent Pre-PCR-Buffer containing Tris-EDTA pH 7.4 and 2-pyrrolidone followed by one wash with 200 µl Rinse solution of water, tween-20, Tris-EDTA and 2-pyrrolidone. These washes remove unligated adapter oligos. Then 60 µl of 95 °C RNAse-free H₂O is added to each filter well. This is allowed to sit for 30 seconds. Suction is used to filter eluant into PCR strips. The plate with the remaining particles is covered and stored at 4 °C until needed post-PCR.

### Amplification

PCR is performed by adding 30 µl of the eluant from the previous step to 20 µl PCR mastermix. This PCR mastermix consists of PCR Buffer a forward primer, a reverse primer, dNTPs, and Pyrophage Exo(-) polymerase enzyme.

Forward primer sequences include: /5Cy5/GCTAGTCCTATGCAATGTCAT (SEQ ID NO: 10), /5Cy5/GCTAGTCCTATGCAATGTCATAAA (SEQ ID NO: 11), /5Cy5/CCTATGCAATGTCATAAATATAAAT (SEQ ID NO: 12), /5Cy5/GGCTGAGTGCAGTGCGAGAAA (SEQ ID NO: 13), and /5Cy5/GGTTGGCCACGTGACTTGATCTT (SEQ ID NO: 14).

Reverse primer sequences include: GATAGATCCGCTTATCGACG (SEQ ID NO: 15), GATAGATCCGCTTATCGACGGAT (SEQ ID NO: 16), CGCTTATCGACGGATATATTTTATTA (SEQ ID NO: 17), CGCTTATCGACGGATATATTTTAAA (SEQ ID NO: 18), CGCTTATCGACGGATATATTTTATTA (SEQ ID NO: 19) and TCGCGCTAATTGACGCTT (SEQ ID NO: 20).

Alternatively, the mastermix may replace 75%-25% of the dTTP with dUTP, and 1ul of H2O will be replaced with 1 µl COD-UNG (Arcticzymes). The PCR mastermix and eluant is thoroughly mixed, and a thermocycler is used to amplify the target with standard PCR cycling.

### Recapture

Immediately after the PCR reaction is completed, the filter plate containing the hydrogel particles is brought back to room temperature and 100 µl Re-Hybridization buffer (consisting of 1M NaCl, 5x TE pH 8.0, 50% 2-pyrrolidone) is added to each well containing particles, and 40 µl of the PCR product is added as well. This mixture is shaken for 30 minutes at 37 °C to allow hybridization of the labeled product to the particles. After hybridization the particles are washed twice with 200 µl of a Rinse Solution. Finally, 175 µl of density matched Run Buffer is added to each well, and the sample is run on an appropriate flow cytometer.

### Scanning and Analysis

Final amplicon measurement can be carried out by scanning encoded microparticles through a flow cytometer (such as a Guava 8HT flow cytometer), or by deconvoluting fluorescent codes on standard instrumentation such as a microarray reader.

### Results

### Limit of Detection with Synthetic microRNAs

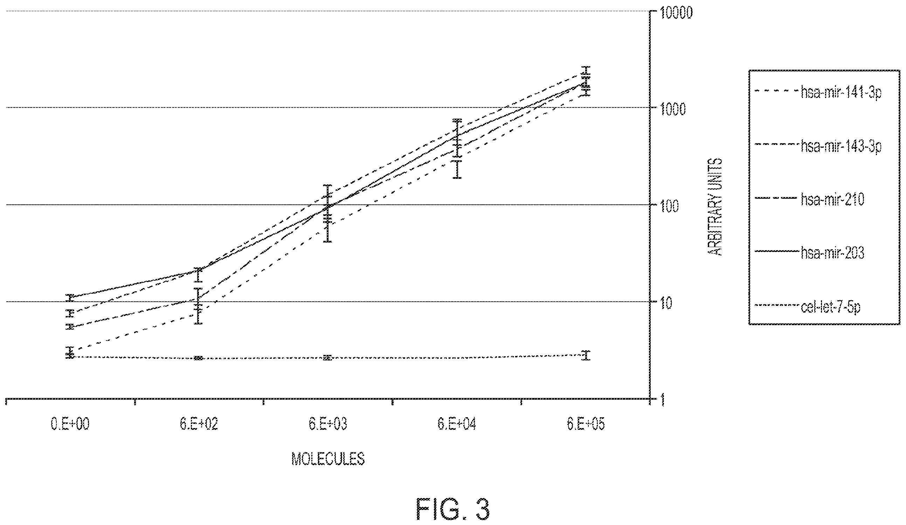

Synthetic microRNA targets, assayed at known concentrations, were used to assess the absolute performance of this multiplexed PCR-coupled hybridization assay. The data below in Figure 3 demonstrate that the limit of detection of this assay may be as low as 100 molecules per sample at the cycling conditions used.

### Tunable Endpoint Assay

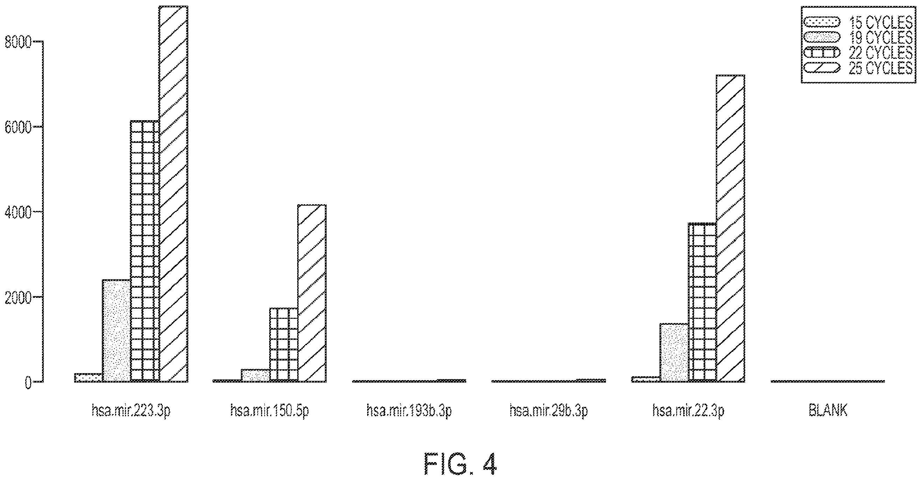

The degree of amplification from the multiplexed PCR-coupled hybridization assay can be tuned via cycle number. The data in Figure 4 show signal for three detected targets and three undetected targets with increasing cycle number. This shows that the sensitivity and dynamic range covered by this assay can be shifted as needed.

### Cross-Platform Comparison

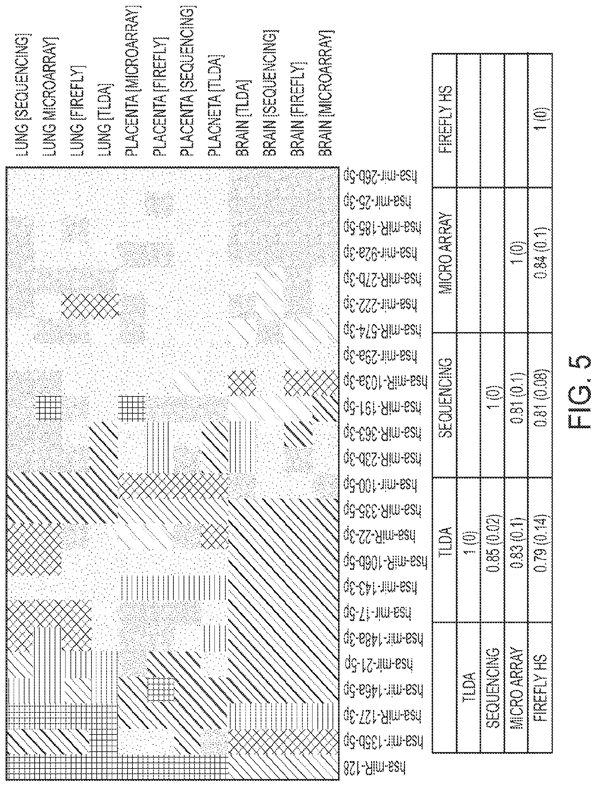

The multiplexed PCR-coupled microRNA assay, referred to below as Firefly HS", was used to measure microRNA profiles across RNA isolated from three tissue types: lung, brain, and placental. These results were directly compared to profiles resulting from RNA-Seq on the Illumina platform, Taqman qPCR (TLDA card format), and microarray analysis (see Figure 5). Triplicate measurements demonstrate robust profiles that cluster well between the different analysis methods used. The Pearson correlations between each method are shown in Figure 5.

### Input Amount

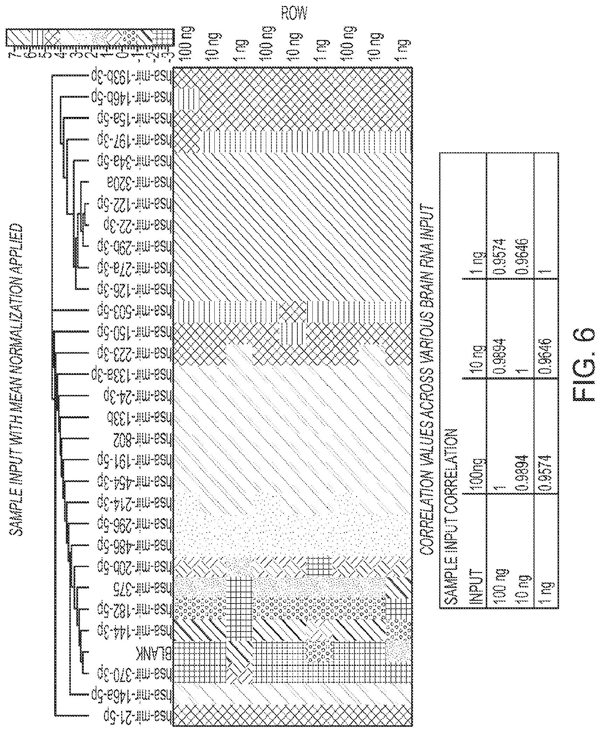

Total RNA isolated from brain tissue was assayed with the PCR-coupled hybridization assay across two logs of total RNA input. As demonstrated in Figure 6, at 100 nanograms, 10 nanograms, and 1 nanogram of total RNA input, robust profiles were obtained for the panel of selected microRNA targets. Signal magnitudes were lower for reduced RNA input. However, microRNA profiles were virtually identical when mean normalization was applied. R-squared correlations across various sample inputs were found to between 0.9574 and 0.9894, indicating a high level of agreement for the various levels of RNA input.

### microRNA Profiling from Serum RNA

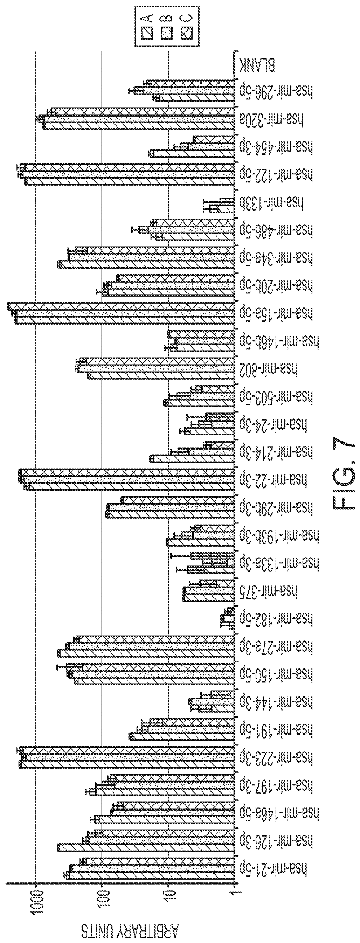

RNA isolated from human serum was assayed in triplicate for 30 microRNA targets with the novel PCR-coupled assay. The results shown in Figure 7 demonstrate that this assay is well-suited for measuring the abundance of small RNA molecules in serum samples, as almost all targets microRNAs were detected from these samples.

### mRNA Profiling Directly from Serum

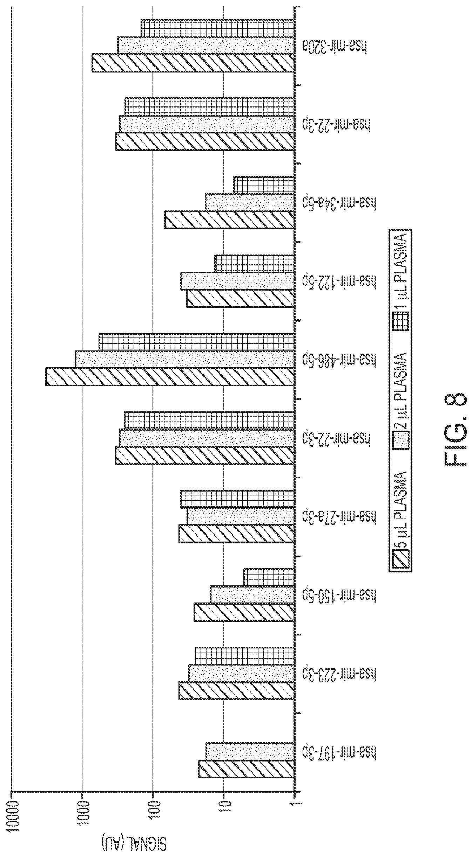

A major barrier to the widespread adoption of circulating nucleic acid biomarkers in clinical settings remains the time and labor associated with organic extraction of RNA from tissues and biofluids. This remains a requirement of most contemporary assays due to the contaminants and potent PCR inhibitors that must be removed via extraction. This novel PCR-coupled hybridization assay utilizes non-fouling hydrogel particles to selectively capture small RNA targets even from a complex sample. Therefore, this method can take crude or minimally purified sample as input. In order to test this concept, a buffer containing proteinase K, a surfactant, and a chaotropic salt was added directed to varying amounts of serum. This buffer serves to disrupt RNA-associated proteins and exosomes as well as to inhibit the activity of RNA-degrading enzymes. As shown in Figure 8, the data obtained from the assay suggest that robust microRNA measurements can be made from as little as 1 microliter of crude serum. Figure 8 shows the microRNA measurements made from 5 microliters, 2 microliters, and 1 microliter of crude serum input. This demonstrates that the assay can be used to detect circulating microRNA markers even from minimally purified samples.

### Example 2. mRNA Capture and Signal Amplification

This example demonstrates that exemplary particles can capture mRNA and that their signal can be amplified via PCR or a PCR-like reaction and the creation of a fluorescent product. Exemplary methods are described below.

mRNA molecules are critical protein-encoding transcripts. These RNAs are typically kilobases long, and present at relatively low copy number compared to other transcript molecules. A highly sensitive multiplexed method is needed in order to measure the expression of these gene transcripts, due to their low copy number and high degradation rates, and the fact that humans alone have more than 20,000 protein-coding mRNAs. The method described above can be modified to capture and measure these and other longer transcription products. Again, a multiplex of solid or semisolid supports, each bearing a unique capture probe can be utilized to capture each mRNA target of interest. A ligation reaction will be used to adapt the 5'-end of each captured mRNA molecule with a short universal oligonucleotide. A multiplexed PCR reaction, making use of a fluorescent poly-T forward primer and the ligated universal reverse priming site, can be used to specifically amplify the entire bound mRNA molecule. By biasing the reaction toward single-stranded product formation, it will be possible to generate many fluorescently-tagged single-stranded copies of the original captured mRNA targets. The use of a PCR polymerase with reverse transcription activity will be necessary in order to amplify the adapted RNA molecule. This has the advantage of using a single primer pair to amplify the signal from a multiplex of mRNA targets.

Figure 9 illustrates the detection of mRNA with capture, modification, and amplification using a poly(T) primer. A single adapter is ligated to the 5' end of the mRNA species, and universal amplification is performed with a primer sequence within the adapter region and one that contains a poly(T) region to prime against the poly(A) mRNA tail.

Alternatively, this method could make use of hydrogel microparticles that each contain a single mRNA-specific primer instead of a mRNA-specific probe. This would lead to a one-step reaction, in which primer-laden particles take place directly in the PCR reaction. The final double-stranded PCR amplicon will, by necessity, be mostly attached to the hydrogel microparticles at the conclusion of PCR cycling.

At the conclusion of these multiplexed mRNA assays, the signal generated by each bound mRNA target molecule can be measured by scanning the encoded microparticle library through a flow cytometer. Alternatively, a microarray reader or fluorescent microscope may be used in order to assess the signal from each particle type.

### Example 3. IncRNA Detection and Signal Amplification

This example demonstrates that exemplary particles can capture IncRNA and that their signal can be amplified via PCR or a PCR-like reaction and the creation of a fluorescent product. Exemplary methods are described below.

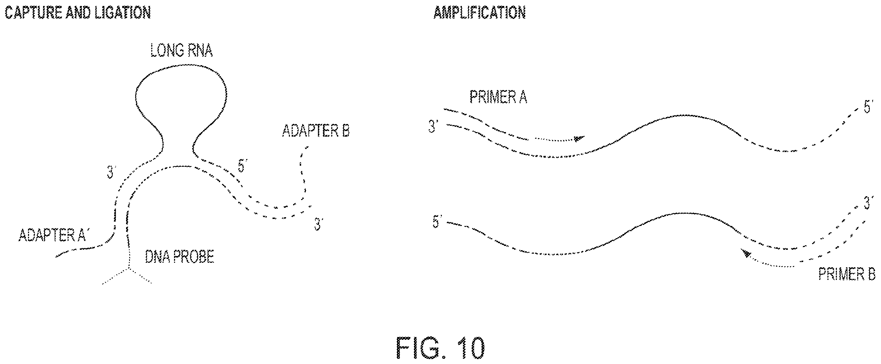

Additional longer non-coding RNA transcripts, including lincRNAs, are of increasing interest to life science researchers and clinical molecular researchers. The method described above, for microRNA and other small-RNA targets, may be adapted for longer RNA targets. These targets will necessitate an alternative probe design, as synthetic oligonucleotides larger than 100 nucleotides quickly become complex and expensive to manufacture. As illustrated in Figure 10, through capturing both the 3' and 5' ends of a target long-RNA and leaving much of the RNA target in a loop or globule conformation, it is possible to ligate universal oligonucleotide adapters to each end of a longer transcript. The reaction could then proceed as previously described. A single universal forward primer and a single fluorescent reverse primer can be used to amplify a multiplex of captured transcripts without increasing the reaction complexity. This method could be combined with Example 1, such that both large and small transcript species are detected in the same reaction. Final assay readout can be performed by scanning the original encoded capture particles, containing transcript-specific probes in one or multiple defined regions, through a flow cytometer. Alternatively, the encoded particles can be decoded, and the target signals measured, by an array scanner or fluorescent microscope.

### Example 4. Nucleic Acid Quantization Using Nuclease Digestion

This example demonstrates that nuclease digestion can be used in nucleic acid quantization in conjunction with the present invention. Exemplary methods are described below.

Nucleases are enzymes that digest nucleic acids via cleavage of phosphodiester bonds. These enzymes display a wide variety of activity, showing specificity for RNA or DNA, for single-stranded or double-stranded species, and for sites in the middle or at the end of nucleic acid substrates. Because of the specificity in their activity, nucleases can be used effectively in nucleic acid preparation and/or detection. One example for detection is the nuclease protection assay. In this method, (1) a fluorescently-labeled probe oligonucleotide, complementary to a sequence of interest, is incubated with a sample, (2) a nuclease is then used to digest any region of the target or probe that is not double-stranded, and (3) the resulting digest product is run on an electrophoresis gel where fluorescent bands indicate the presence and quantity of the sequence of interest.

The use of nucleases with our assay would allow for the quantization of oligonucleotide sequences internal to longer targets. First, a sample is contacted with the particles and the target is hybridized to probes that are sufficiently complementary. Next, the sample is subjected to nuclease digestion with a nuclease, where the single-stranded ends of the target RNA or DNA are digested leaving only the double-stranded sequence of interest bound to the probe. Then, adapters are ligated to the ends of the digested target and the modified target is subjected to PCR amplification with fluorescent primers. The amplicons are then hybridized to particles and the particles are analyzed for fluorescence to quantify targets.

This method is shown in Figure 11. This approach could be used for the detection of virtually any nucleic acid species, including mRNA, genomic DNA, IncRNA, etc. In this exemplary method, probes would be designed to contain target-specific sequences flanked by sequences specific for universal adapters. In some cases, it may also be beneficial to functionalize the 3' end of the probe with a 3' phosphorylation, inverted dT, or similar modification to protect it from nuclease digestion. In addition, probes may be modified internally to avoid digestion by endonucleases. Probes may also be DNA or RNA, selected to avoid digestion by substrate-specific nucleases. If necessary, adapter sites on the probes may be protected by hybridizing the adapter sequences or other complementary sequences to the probe prior to or during digestion. Several exo- or endonucleases may be used, depending on if the species to be detected is RNA or DNA. Some examples include S1 nuclease, P1 nuclease, RNAse A, RNAseT1, nuclease BAL31, RNAse II, exoribonuclease I, or any combination thereof.

### Example 5. Ultrasensitive ELISA

This example demonstrates that the exemplary assay described in Example 1 can be adapted for protein detection assays. Exemplary methods are described below.

Cytokine assays and other protein detection assays often make use of antibody pairs that specifically target two different epitopes on a target protein. These methods may be adapted in order to utilize the method described above. A multiplex mixture of encoded solid or semi-solid capture particles, each bearing a capture antibody and a specific oligonucleotide sequence will be mixed with the biological sample of interest. The protein target, once captured, will be labeled with the second protein-specific detection antibody functionalized with a specific detection oligonucleotide. This detection oligonucleotide will contain two primer-specific sequences. The detection oligonucleotide can be specifically amplified via PCR or a PCR-like reaction, leading to the formation of significant fluorescent DNA amplicon. The amplicon will be specifically captured by a particle-bound oligonucleotide in a distinct region of a new capture particle or the original capture particle, leading to a significant increase in fluorescent intensity resulting from each bound protein molecule. This method is unique from other PCR-coupled immunoassays in that the assay can be highly multiplexed in a single well. As described above, the assay readout can be performed on a flow cytometer, or some other instrument capable or fluorescence identification of individual species within the multiplexed mixture.

### Example 6. Sequencing Library Construction and Size Selection

This example demonstrates that the exemplary assay described in Example 1 can be used in the construction of a sequencing library of a controlled size. Exemplary methods are described below.

Library construction and sample preparation remain two major hurdles in the widespread clinical adoption of sequencing. The method described above can be adapted in order to generate libraries of controlled size, each bearing the desired platform-specific PCR adapters and/or sequence barcodes for sample pooling. This method is appropriate for generating libraries from either fragmented DNA or directly from RNA.

In this embodiment, a multiplex mixture of solid or semi-solid supports is mixed directly with the biological sample of interest. The solid or semi-solid supports bearing oligonucleotide probes, with random sequences or target specific sequences with adapter-specific regions on the 3' and 5' end of the capture probe, are mixed with the biological samples of interest. Post-capture, a sticky-end or blunt-end ligation step will be performed with a specific ligase enzyme. This is followed by a limited PCR or PCR-like reaction, which amplifies the bound molecules but retains the relative abundance of the target species.

For preparations of RNA, a polymerase with reverse transcription activity, or a reverse transcriptase must be used, in order to convert the bound RNA-DNA hybrid into a DNA amplicon product. Following the limited PCR cycling, the amplified product can be collected, quantified, and used directly as input for sequencing sample preparation. This includes reactions such as emulsion PCR (Ion Torrent PGM™, Ion Torrent Proton™, Roche 454™) or bridge PCR (Illumina™). By altering primer design and adapter design, libraries can be modified to work on any of these sequencing platforms, or to allow sample multiplexing through specific barcode sequence inclusion in the nucleic acid adapters. This method may provide size selection through hydrogel pore size and capture oligonucleotide design, as well as allowing direct library preparation from RNA, or from samples with limited starting material without a specific reverse transcription reaction. Additionally, due to the bioinert or nonfouling properties of some hydrogel substrates (Polyethylene glycol, alginate, chitosan, polylactic/glycolic acid), these substrates can be used to capture nucleic acids from unpurified or minimally purified biological samples.

### Example 7: Exemplary Uses of Assay

This example demonstrates that the exemplary assay described in Example 1 can be used for many specific purposes, including the detection of pathogen DNA or RNA, detection of species in environmental samples, detection of food contaminants, detection of genetic variants, and high-throughput screening of compounds for the pharmaceutical industry.

### Detection of Pathogen DNA or RNA

It is often advantageous to detect low levels of DNA or RNA from a particular pathogen in a sample, be it for biosafety, epidemiological or diagnostic purposes. The method disclosed herein can be utilized for this purpose as follows: 1) A free-floating probe specific to the target sequence is hybridized with the nucleic acids derived from the sample. 2) Adapters are ligated to both sides as described, but with adapter sequences designed to match the target DNA near the ligation sites. 3) Amplification is performed as described above, and 4) The amplified products are captured on hydrogel particles for multiplex detection. Probes can be specific to a particular pathogen species, or entire evolutionary clades, depending on the degree of conservation of the targeted sequence among related pathogens.

### Detection of Species in Environmental Samples

Similarly as in the detection of pathogens, the method disclosed herein can be used to detect small amounts of a particular species or clades of related species in environmental samples for purposes of ecological monitoring or ecological research.

### Detection of Food Contaminants

Similarly as in the detection of pathogens, the method disclosed herein can be used to detect small amounts of a particular species or clades of related species in food samples for monitoring of food safety and labeling accuracy.

### Detection of Genetic Variants

The embodiment described above regarding pathogen detection is sensitive to single base differences at the ligation sites. This makes it suitable for detecting single nucleotide polymorphisms in biological samples, for medical, forensic, agricultural, and ecological purposes. Any other polymorphism could also be detected. Polymorphisms can be detected at low levels, which is important in oncological applications, where a small number of aberrant cells may be masked by many more normal cells.

## Claims

1. A method of detecting target nucleic acid, comprising steps of:
a) Contacting a sample with a first set of particles bearing a plurality of capturing probes, each comprising at least one target capturing sequence, under conditions that permit the plurality of capturing probes to capture one or more target nucleic acids in the sample;
b) Amplifying the captured one or more target nucleic acids in a reaction mixture comprising a detectable entity such that the amplified one or more target nucleic acids are labeled with the detectable entity;
c) Incubating amplification product with a second set of particles bearing a plurality of re-capturing probes such that the amplified one or more target nucleic acids are re-captured by the plurality of the re-capturing probes, wherein the first set and second set of particles are identical;
d) Detecting signal generated by a detectable entity associated with the re-captured amplified one or more target nucleic acids, wherein the presence and/or abundance of the detectable signal indicates the presence and/or abundance of the one or more target nucleic acids in the sample.

2. The method of claim 1, wherein the capturing probes and re-capturing probes:
(a) comprise (i) one target capturing sequence and bind specifically to one distinct target nucleic acid, or (ii) multiple distinct target capturing sequences and bind to multiple distinct target nucleic acids; and/or
(b) contain one or more mismatch bases against the one or more target nucleic acids.

3. The method of claim 1 or claim 2, wherein the particles are made of a material selected from the group consisting of hydrogel, glass, photoresists, silica, polystyrene, polyethylene glycol, agarose, chitosan, alginate, PLGA, optical fiber, cellulose, and a combination thereof, optionally wherein the material is hydrogel.

4. The method of any one of the preceding claims, wherein the capturing and recapturing probes are embedded throughout one or more probe regions of the particle, optionally wherein the particle further comprises one or more encoding regions and wherein the one or more encoding regions bear detectable moieties that give the identity of the capturing and re-capturing probes.

5. The method of any one of the preceding claims, wherein the one or more target nucleic acids are microRNAs, mRNAs, non-coding transcripts, genomic DNA, cDNAs, siRNAs, DNA/RNA chimera, or a combination thereof.

6. The method of any one of the preceding claims, wherein the probe is DNA, RNA, DNA/RNA chimera, or a combination thereof, optionally wherein the probe is specific to the target nucleic acid and comprises a target capture sequence that is substantially complementary to the target nucleic acid.

7. The method of any one of the preceding claims, wherein the method further comprises a step of coupling one or more adapters to the captured target nucleic acid, optionally wherein the one or more adapters:
(a) are universal adapters, optionally wherein the one or more adapters are coupled to the target nucleic acid at the 3'-terminus, the 5'-terminus, or both the 3'-terminus and 5'-terminus; and/or
(b) are DNA, RNA, DNA/RNA chimera, or a combination thereof; and/or
(c) are coupled to the target nucleic acid via ligation, optionally wherein the ligation is performed by a DNA or RNA ligase enzyme; and/or
(d) comprise sequences specifically designed to serve as sites for polymerase chain reaction priming, reverse transcription, or modification by other DNA-modifying or RNA-modifying enzymes.

8. The method of any one of the preceding claims, wherein the captured target nucleic acid is first digested by a nuclease or restriction enzyme to remove single-stranded 5' and or 3' regions prior to the coupling of the one or more adapters, and/or wherein each of the capturing and recapturing probes further comprises sequences complementary to the one or more adapters, optionally wherein the sequences complementary to the one or more adapters are adjacent to the target capture sequence.

9. The method of any one of the preceding claims, wherein
(i) the step of amplifying the captured target nucleic acid comprises performing a polymerase chain reaction (PCR), optionally wherein the PCR reaction uses polymerase enzyme selected from Taq, Bst, and/or Phi29, optionally wherein the PCR is performed
(a) with a single primer set, optionally wherein the PCR is performed with one primer; and/or
(b) with primers attached to the particle; and/or
(c) using a combination of universal, specific, or poly(A) primers; and/or
(ii) the captured target is reverse transcribed prior to amplification, optionally wherein reverse transcription is catalyzed by a polymerase enzyme with reverse transcriptase activity, optionally wherein the polymerase enzyme is Pyrophage or TtH, optionally wherein reverse transcription is catalyzed by one enzyme and PCR amplification is carried out by a second enzyme; and/or
(iii) the step of amplifying the captured target nucleic acid is performed isothermally; and/or
(vi) the target nucleic acid and/or the one or more adapters are circularized via ligation or enzymatic polymerization.

10. The method of any one the preceding claims, wherein the detectable entity is (i) selected from the group consisting of fluorophores, dye, biotin, radioisotopes, antibodies, aptamers, polypeptides, quantum dots, chromophores; and/ or (ii) provided in the reaction mixture as labeled primer, labeled dNTPs and/or intercalating dye.

11. The method of any one of the preceding claims, wherein
(i) the captured one or more target nucleic acids are separated from the capturing probes prior to amplification, optionally wherein the captured one or more target nucleic acids are separated from the capturing probes by denaturation using heat, chemical denaturants, or a helicase enzyme; and/or
(ii) the particle is present during the time of amplification; and/or
(iii) the step of amplifying the captured one or more target nucleic acids is performed using a single primer or less than 5 primer pairs.

12. The method of any one of claims 9-11, wherein the PCR is biased such that a substantial fraction of the amplified one or more target nucleic acids is single-stranded, optionally wherein the PCR is biased towards single-stranded amplified target nucleic acid through (i) designing a forward primer with a significantly lower annealing temperature than a reverse primer, or (ii) adding the forward primer at a concentration such that it is exhausted during the PCR reaction, optionally wherein the ratio between the forward primer and the reverse primer is less than 1:2.

13. The method of any one of the preceding claims, wherein
(i) the amplification product and the plurality of re-capturing probes are incubated under stringent hybridization condition; and/or
(ii) the re-capturing of amplified one or more target nucleic acids is performed under substantially more stringent conditions than the capturing step; and/or
(iii) the particle is rinsed between steps to remove unbound probes, target nucleic acids and/or adapters; and/or
(iv) the reaction mixture comprises (a) a single primer set used to amplify multiple distinct target nucleic acids or (b) multiple primer sets used to amplify multiple distinct target nucleic acids; and/or
(v) the conditions are tuned in order to give stringent capture by controlling: temperature, time, monovalent salt concentration, divalent salt concentration, dNTP concentration, or the addition of DMSO, formamide, polyethylene glycol, 2-pyrrolidone, or other agents that alter the kinetics of DNA duplex formation.

14. The method of any one of the preceding claims, wherein the sample is a biological sample, optionally wherein the biological sample is a preparation of isolated DNA or RNA, protease tissue digest, cell lysate, serum, plasma, whole blood, urine, stool, saliva, cord blood, chorionic villus sample, chorionic villus sample culture, amniotic fluid, amniotic fluid culture, transcervical lavage fluid, or a combination thereof.

15. The method of any one of the preceding claims, wherein the signal generated by the detectable entity is detected by a flow cytometer, or array scanner, optionally wherein the signal is quantified.

16. The method of any one of the preceding claims, wherein the plurality of capturing and recapturing probes comprises multiple probes specific to multiple target nucleic acids, wherein the multiple probes are associated with multiple particles, with each particle comprising probes specific to same target nucleic acid, optionally wherein (i) the each particle is encoded to provide identity of the specific probes thereon, wherein the each particle is encoded through incorporation of one or more fluorophores with known spectral characteristics; or (ii) the multiple probes are located on multiple distinct regions of the particle.

17. The method of any one of the preceding claims, wherein each target nucleic acid is present at low abundance in the sample, optionally wherein each target nucleic acid represents less than 1%, or less than 0.1%, less than 1 out of a million, or less than 1 out of 10 million of total nucleic acids in the biological sample.

18. A diagnostic method comprising a step of detecting one or more target nucleic acids according to any one of the preceding claims.

## Patentansprüche

1. Verfahren zum Nachweis einer Zielnukleinsäure, umfassend die Schritte:
a) Inkontaktbringen einer Probe mit einem ersten Satz von Partikeln, die eine Vielzahl von Einfangsonden tragen, von denen jede mindestens eine Zieleinfangsequenz umfasst, unter Bedingungen, die es der Vielzahl von Einfangsonden ermöglichen, eine oder mehrere Zielnukleinsäuren in der Probe einzufangen;
b) Amplifizieren der eingefangenen ein oder mehreren Zielnukleinsäuren in einem Reaktionsgemisch, das eine detektierbare Entität umfasst, so dass die amplifizierten ein oder mehreren Zielnukleinsäuren mit der detektierbaren Entität markiert werden;
c) Inkubieren des Amplifikationsprodukts mit einem zweiten Satz von Partikeln, die eine Vielzahl von Wiedereinfangsonden tragen, so dass die amplifizierten ein oder mehreren Zielnukleinsäuren von der Vielzahl von Wiedereinfangsonden erneut eingefangen werden, wobei der erste Satz und der zweite Satz von Partikeln identisch sind;
d) Detektieren eines Signals, das von einer detektierbaren Entität erzeugt wird, die mit den erneut eingefangenen amplifizierten ein oder mehreren Zielnukleinsäuren assoziiert ist, wobei das Vorhandensein und/oder die Häufigkeit des detektierbaren Signals das Vorhandensein und/oder die Häufigkeit der ein oder mehreren Zielnukleinsäuren in der Probe anzeigt.

2. Verfahren nach Anspruch 1, wobei die Einfangsonden und die Wiedereinfangsonden:
(a) (i) eine Zieleinfangsequenz umfassen und spezifisch an eine gesonderte Zielnukleinsäure binden, oder (ii) mehrere gesonderte Zieleinfangsequenzen umfassen und an mehrere gesonderte Zielnukleinsäuren binden; und/oder
(b) eine oder mehrere Fehlpaarungsbasen gegen die eine oder die mehreren Zielnukleinsäuren enthalten.

3. Verfahren nach Anspruch 1 oder Anspruch 2, wobei die Partikel aus einem Material hergestellt sind, das aus der Gruppe ausgewählt ist, die aus Hydrogel, Glas, Photoresists, Siliciumdioxid, Polystyrol, Polyethylenglycol, Agarose, Chitosan, Alginat, PLGA, optischen Fasern, Cellulose und einer Kombination davon besteht, wobei das Material optional ein Hydrogel ist.

4. Verfahren nach einem beliebigen der vorhergehenden Ansprüche, wobei die Einfangsonden und Wiedereinfangsonden überall in einer oder mehreren Sondenregionen des Partikels eingebettet sind, wobei das Partikel optional ferner eine oder mehrere Kodierungsregionen umfasst und wobei die eine oder die mehreren Kodierungsregionen detektierbare Einheiten tragen, die die Identität der Einfangsonden und Wiedereinfangsonden angeben.

5. Verfahren nach einem beliebigen der vorhergehenden Ansprüche, wobei es sich bei den ein oder mehreren Zielnukleinsäuren um mikroRNAs, mRNAs, nicht-kodierende Transkripte, genomische DNA, cDNAs, siRNAs, DNA/RNA-Chimären oder eine Kombination davon handelt.

6. Verfahren nach einem beliebigen der vorhergehenden Ansprüche, wobei die Sonde DNA, RNA, DNA/RNA-Chimäre oder eine Kombination davon ist, wobei die Sonde optional spezifisch für die Zielnukleinsäure ist und eine Zieleinfangsequenz umfasst, die im Wesentlichen komplementär zu der Zielnukleinsäure ist.

7. Verfahren nach einem beliebigen der vorhergehenden Ansprüche, wobei das Verfahren ferner einen Schritt des Koppelns eines oder mehrerer Adapter an die eingefangene Zielnukleinsäure umfasst, wobei optional der eine oder die mehreren Adapter:
(a) universelle Adapter sind, wobei optional der eine oder die mehreren Adapter an die Zielnukleinsäure am 3'-Terminus, am 5'-Terminus oder sowohl am 3'-Terminus als auch am 5'-Terminus gekoppelt sind; und/oder
(b) DNA, RNA, DNA/RNA-Chimäre oder eine Kombination davon sind; und/oder
(c) über Ligation an die Zielnukleinsäure gekoppelt werden, wobei die Ligation optional durch ein DNA- oder RNA-Ligaseenzym durchgeführt wird; und/oder
(d) Sequenzen umfassen, die speziell zum Fungieren als Stellen für das Priming der Polymerasekettenreaktion, der reversen Transkription oder der Modifikation durch andere DNA-modifizierende oder RNA-modifizierende Enzyme entworfen sind.

8. Verfahren nach einem beliebigen der vorhergehenden Ansprüche, wobei die eingefangene Zielnukleinsäure zuerst durch eine Nuklease oder ein Restriktionsenzym verdaut wird, um einzelsträngige 5'- und/oder 3'-Regionen vor der Kopplung des einen oder der mehreren Adapter zu entfernen, und/oder wobei jede der Einfang- und Wiedereinfangsonden ferner Sequenzen umfasst, die zu dem einen oder den mehreren Adaptern komplementär sind, wobei die Sequenzen, die zu dem einen oder den mehreren Adaptern komplementär sind, optional zu der Zieleinfangsequenz benachbart sind.

9. Verfahren nach einem beliebigen der vorhergehenden Ansprüche, wobei
(i) der Schritt des Amplifizierens der eingefangenen Zielnukleinsäure das Durchführen einer Polymerasekettenreaktion (PCR) umfasst, wobei optional die PCR-Reaktion ein Polymeraseenzym verwendet, das aus Taq, Bst und/oder Phi29 ausgewählt ist, wobei optional die PCR durchgeführt wird
(a) mit einem einzelnen Primersatz, wobei optional die PCR mit einem Primer durchgeführt wird; und/oder
(b) mit an das Partikel gebundenen Primern; und/oder
(c) unter Verwenden einer Kombination von universellen, spezifischen oder Poly(A)-Primern; und/oder
(ii) das eingefangene Ziel vor der Amplifikation revers transkribiert wird, wobei optional die reverse Transkription durch ein Polymeraseenzym mit Reverse-Transkriptase-Aktivität katalysiert wird, wobei optional das Polymeraseenzym Pyrophage oder TtH ist, wobei optional die reverse Transkription durch ein Enzym katalysiert wird und die PCR-Amplifikation durch ein zweites Enzym ausgeführt wird; und/oder
(iii) der Schritt des Amplifizierens der eingefangenen Zielnukleinsäure isotherm durchgeführt wird; und/oder (vi) die Zielnukleinsäure und/oder der eine oder die mehreren Adapter durch Ligation oder enzymatische Polymerisation zirkularisiert werden.

10. Verfahren nach einem beliebigen der vorhergehenden Ansprüche, wobei die detektierbare Entität (i) ausgewählt ist aus der Gruppe bestehend aus Fluorophoren, Farbstoff, Biotin, Radioisotopen, Antikörpern, Aptameren, Polypeptiden, Quantenpunkten, Chromophoren; und/oder (ii) im Reaktionsgemisch als markierter Primer, markierte dNTPs und/oder ein interkalierender Farbstoffe bereitgestellt wird.

11. Verfahren nach einem der vorhergehenden Ansprüche, wobei
(i) die eingefangenen ein oder mehreren Zielnukleinsäuren vor der Amplifikation von den Einfangsonden getrennt werden, wobei optional die eingefangenen ein oder mehreren Zielnukleinsäuren von den Einfangsonden durch Denaturierung unter Verwendung von Wärme, chemischen Denaturierungsmitteln oder einem Helikaseenzym getrennt werden; und/oder
(ii) das Partikel während der Zeit der Amplifikation anwesend ist; und/oder
(iii) der Schritt des Amplifizierens der eingefangenen ein oder mehreren Zielnukleinsäuren unter Verwendung eines einzelnen Primers oder weniger als 5 Primerpaaren durchgeführt wird.

12. Verfahren nach einem beliebigen der Ansprüche 9-11, wobei die PCR derart vorgeneigt ist, dass ein wesentlicher Anteil der amplifizierten ein oder mehreren Zielnukleinsäuren einzelsträngig ist, wobei die PCR optional gegen einzelsträngige amplifizierte Zielnukleinsäuren vorgeneigt ist durch (i) Entwerfen eines Vorwärtsprimers mit einer signifikant niedrigeren Annealingtemperatur als ein Rückwärtsprimer oder (ii) Hinzufügen des Vorwärtsprimers bei einer solchen Konzentration, dass er während der PCR-Reaktion aufgebraucht wird, wobei optional das Verhältnis zwischen Vorwärtsprimer und Rückwärtsprimer kleiner als 1:2 ist.

13. Verfahren nach einem beliebigen der vorhergehenden Ansprüche, wobei
(i) das Amplifikationsprodukt und die Vielzahl von Wiedereinfangsonden unter stringenten Hybridisierungsbedingungen inkubiert werden; und/oder
(ii) das erneute Einfangen einer oder mehrerer amplifizierter Zielnukleinsäuren unter wesentlich stringenteren Bedingungen als im Einfangschritt durchgeführt wird; und/oder
(iii) das Partikel zwischen den Schritten gespült wird, um ungebundene Sonden, Zielnukleinsäuren und/oder Adapter zu entfernen; und/oder
(iv) das Reaktionsgemisch (a) einen einzelnen Primersatz, der zur Amplifikation mehrerer gesonderter Zielnukleinsäuren verwendet wird, oder (b) mehrere Primersätze, die zur Amplifikation mehrerer gesonderter Zielnukleinsäuren verwendet werden, umfasst; und/oder
(v) die Bedingungen eingestellt werden, damit ein stringentes Einfangen erreicht wird, durch Steuern von: Temperatur, Zeit, Konzentration einwertiger Salze, Konzentration zweiwertiger Salze, dNTP-Konzentration oder die Zugabe von DMSO, Formamid, Polyethylenglykol, 2-Pyrrolidon oder anderen Mitteln, die die Kinetik der DNA-Duplex-Bildung verändern.

14. Verfahren nach einem beliebigen der vorhergehenden Ansprüche, wobei die Probe eine biologische Probe ist, wobei es sich bei der biologischen Probe optional um ein Präparat aus isolierter DNA oder RNA, Protease-Gewebeverdau, Zelllysat, Serum, Plasma, Vollblut, Urin, Stuhl, Speichel, Nabelschnurblut, Chorionzottenprobe, Chorionzottenprobenkultur, Fruchtwasser, Fruchtwasserkultur, transzervikale Spülflüssigkeit oder eine Kombination davon handelt.

15. Verfahren nach einem beliebigen der vorhergehenden Ansprüche, wobei das von der detektierbaren Entität erzeugte Signal durch ein Durchflusszytometer oder einen Array-Scanner detektiert wird, wobei das Signal optional quantifiziert wird.

16. Verfahren nach einem beliebigen der vorhergehenden Ansprüche, wobei die Vielzahl von Einfang- und Wiedereinfangsonden mehrere Sonden umfasst, die für mehrere Zielnukleinsäuren spezifisch sind, wobei die mehreren Sonden mit mehreren Partikeln assoziiert sind, wobei jedes Partikel Sonden umfasst, die für dieselbe Zielnukleinsäure spezifisch sind, wobei optional (i) jedes Partikel kodiert ist, um die Identität der spezifischen Sonden darauf anzugeben, wobei jedes Partikel durch Einbau eines oder mehrerer Fluorophore mit bekannten spektralen Eigenschaften kodiert wird; oder (ii) die mehreren Sonden auf mehreren gesonderten Regionen des Partikels lokalisiert sind.

17. Verfahren nach einem beliebigen der vorhergehenden Ansprüche, wobei jede Zielnukleinsäure in der Probe in geringer Häufigkeit vorhanden ist, wobei optional jede Zielnukleinsäure weniger als 1% oder weniger als 0,1%, weniger als 1 aus einer Millionen oder weniger als 1 aus 10 Millionen der gesamten Nukleinsäuren in der biologischen Probe repräsentiert.

18. Diagnoseverfahren, umfassend einen Schritt des Nachweises einer oder mehrerer Zielnukleinsäuren nach einem beliebigen der vorhergehenden Ansprüche.

## Revendications

1. Procédé de détection d'acide nucléique cible, comprenant les étapes consistant à :
a) mettre en contact un échantillon avec un premier ensemble de particules portant une pluralité de sondes de capture, chacune comprenant au moins une séquence de capture de cible, dans des conditions qui permettent à la pluralité de sondes de capture de capturer un ou plusieurs acides nucléiques cibles présents dans l'échantillon ;
b) amplifier le ou les acides nucléiques cibles capturés dans un mélange réactionnel comprenant une entité détectable de façon telle que le ou les acides nucléiques cibles amplifiés sont marqués avec l'entité détectable ;
c) incuber le produit d'amplification avec un second ensemble de particules portant une pluralité de sondes de recapture de façon telle que le ou les acides nucléiques cibles amplifiés sont recapturés par la pluralité des sondes de recapture, le premier ensemble et le second ensemble de particules étant identiques ;
d) détecter un signal produit par une entité détectable associée à l'acide nucléique cible amplifié recapturé ou aux acides nucléiques cibles amplifiés recapturés, la présence et/ou l'abondance du signal détectable indiquant la présence et/ou l'abondance du ou des acides nucléiques cibles dans l'échantillon.

2. Procédé selon la revendication 1, dans lequel les sondes de capture et les sondes de recapture :
(a) comprennent
(i) une séquence de capture de cible et se lient spécifiquement à un acide nucléique cible distinct ou
(ii) de multiples séquences de capture de cible distinctes et se lient à de multiples acides nucléiques cibles distincts ; et/ou
(b) contiennent une ou plusieurs bases de mésappariement avec le ou les acides nucléiques cibles.

3. Procédé selon la revendication 1 ou la revendication 2, dans lequel les particules sont constituées d'un matériau choisi dans le groupe constitué par un hydrogel, du verre, des résines photosensibles, la silice, le polystyrène, le polyéthylèneglycol, l'agarose, le chitosane, un alginate, le PLGA, une fibre optique, la cellulose et une association de ceux-ci, éventuellement dans lequel le matériau est un hydrogel.

4. Procédé selon l'une quelconque des revendications précédentes, dans lequel les sondes de capture et de recapture sont enchâssées sur toute l'étendue d'une ou plusieurs régions de sondes de la particule, éventuellement la particule comprenant en outre une ou plusieurs régions codantes et la ou les régions codantes portant des fractions détectables qui donnent l'identité des sondes de capture et de recapture.

5. Procédé selon l'une quelconque des revendications précédentes, dans lequel le ou les acides nucléiques cibles sont des microARN, des ARNm, des produits de transcription non codants, de l'ADN génomique, des ADNc, des ARNsi, de l'ADN/ARN chimère ou une association de ceux-ci.

6. Procédé selon l'une quelconque des revendications précédentes, dans lequel la sonde est de l'ADN, de l'ARN, de l'ADN/ARN chimère ou une association de ceux-ci, éventuellement dans lequel la sonde est spécifique pour l'acide nucléique cible et comprend une séquence de capture de cible qui est en grande partie complémentaire à l'acide nucléique cible.

7. Procédé selon l'une quelconque des revendications précédentes, le procédé comprenant en outre une étape consistant à coupler un ou plusieurs adaptateurs à l'acide nucléique cible capturé, éventuellement le ou les adaptateurs :
(a) étant des adaptateurs universels, éventuellement le ou les adaptateurs étant couplés à l'acide nucléique cible à l'extrémité 3', à l'extrémité 5' ou à la fois à l'extrémité 3' et à l'extrémité 5' ; et/ou
(b) étant de l'ADN, de l'ARN, de l'ADN/ARN chimère ou une association de ceux-ci ; et/ou
(c) étant couplés à l'acide nucléique cible par l'intermédiaire d'une ligature, éventuellement la ligature étant réalisée par une enzyme ADN ou ARN ligase ; et/ou
(d) comprenant des séquences spécifiquement conçues pour servir de sites pour l'amorçage d'une réaction en chaîne par polymérase, une transcription inverse ou une modification par d'autres enzymes de modification d'ADN ou de modification d'ARN.

8. Procédé selon l'une quelconque des revendications précédentes, dans lequel l'acide nucléique cible capturé est d'abord digéré par une nucléase ou une enzyme de restriction pour éliminer les régions 5' et/ou 3' simple brin avant le couplage à l'adaptateur ou aux adaptateurs et/ou dans lequel chacune des sondes de capture et de recapture comprend en outre des séquences complémentaires à l'adaptateur ou aux adaptateurs, éventuellement les séquences complémentaires à l'adaptateur ou aux adaptateurs étant adjacentes à la séquence de capture de cible.

9. Procédé selon l'une quelconque des revendications précédentes, dans lequel
(i) l'étape consistant à amplifier l'acide nucléique cible capturé comprend la réalisation d'une réaction en chaîne par polymérase (PCR), éventuellement la réaction de PCR utilisant une enzyme polymérase choisie parmi les enzymes Taq, Bst et/ou Phi29, éventuellement la PCR étant réalisée
(a) avec un seul ensemble d'amorces, éventuellement la PCR étant réalisée avec une amorce ; et/ou
(b) avec des amorces attachées à la particule ; et/ou
(c) à l'aide d'une association d'amorces universelles, spécifiques ou poly(A) ; et/ou
(ii) la cible capturée subit une transcription inverse avant amplification, éventuellement la transcription inverse étant catalysée par une enzyme polymérase à activité de transcriptase inverse, éventuellement l'enzyme polymérase étant l'enzyme PyroPhage ou TtH, éventuellement la transcription inverse étant catalysée par une enzyme et l'amplification par PCR étant effectuée par une seconde enzyme ; et/ou
(iii) l'étape consistant à amplifier l'acide nucléique cible capturé est réalisée de manière isotherme ; et/ou
(vi) l'acide nucléique cible et/ou le ou les adaptateurs sont circularisés par l'intermédiaire d'une ligature ou d'une polymérisation enzymatique.

10. Procédé selon l'une quelconque des revendications précédentes, dans lequel l'entité détectable est
(i) choisie dans le groupe constitué par les fluorophores, un colorant, la biotine, les radioisotopes, les anticorps, les aptamères, les polypeptides, les points quantiques, les chromophores ; et/ou
(ii) fournie dans le mélange réactionnel sous forme d'amorce marquée, de dNTP marqués et/ou de colorant intercalant.

11. Procédé selon l'une quelconque des revendications précédentes, dans lequel
(i) le ou les acides nucléiques cibles capturés sont séparés des sondes de capture avant amplification, éventuellement le ou les acides nucléiques cibles capturés étant séparés des sondes de capture par dénaturation à l'aide de chaleur, de dénaturants chimiques ou d'une enzyme hélicase ; et/ou
(ii) la particule est présente pendant le temps d'amplification ; et/ou
(iii) l'étape consistant à amplifier le ou les acides nucléiques cibles capturés est réalisée à l'aide d'une seule amorce ou de moins de 5 paires d'amorces.

12. Procédé selon l'une quelconque des revendications 9-11, dans lequel la PCR est biaisée de façon telle qu'une fraction importante du ou des acides nucléiques cibles amplifiés est simple brin, éventuellement la PCR étant biaisée en direction d'un acide nucléique cible amplifié simple brin grâce à
(i) la conception d'une amorce sens présentant une température d'hybridation sensiblement plus faible que celle d'une amorce antisens ou
(ii) l'ajout de l'amorce sens en une concentration telle qu'elle est épuisée pendant la réaction de PCR, éventuellement le rapport entre l'amorce sens et l'amorce antisens étant inférieur à 1:2.

13. Procédé selon l'une quelconque des revendications précédentes, dans lequel
(i) le produit d'amplification et la pluralité de sondes de recapture sont incubés dans des conditions d'hybridation stringentes ; et/ou
(ii) la recapture d'un ou plusieurs acides nucléiques cibles amplifiés est réalisée dans des conditions notablement plus stringentes que celles de l'étape de capture ; et/ou
(iii) la particule est rincée entre des étapes pour éliminer les sondes, acides nucléiques cibles et/ou adaptateurs non liés ; et/ou
(iv) le mélange réactionnel comprend
(a) un seul ensemble d'amorces utilisé pour amplifier de multiples acides nucléiques cibles distincts ou
(b) de multiples ensembles d'amorces utilisés pour amplifier de multiples acides nucléiques cibles distincts ; et/ou
(v) les conditions sont spécialement ajustées afin de donner une capture stringente par le réglage de : la température, la durée, la concentration en sels monovalents, la concentration en sels divalents, la concentration en dNTP ou l'ajout de DMSO, de formamide, de polyéthylèneglycol, de 2-pyrrolidone ou d'autres agents qui modifient la cinétique de la formation de duplex d'ADN.

14. Procédé selon l'une quelconque des revendications précédentes, dans lequel l'échantillon est un échantillon biologique, éventuellement l'échantillon biologique étant une préparation d'ADN ou ARN isolé, un produit de digestion de tissu par une protéase, un lysat de cellules, du sérum, du plasma, du sang entier, de l'urine, des selles, de la salive, du sang de cordon ombilical, un échantillon de villosités choriales, une culture d'échantillon de villosités choriales, du liquide amniotique, une culture de liquide amniotique, du liquide de lavage transcervical ou une association de ceux-ci.

15. Procédé selon l'une quelconque des revendications précédentes, dans lequel le signal produit par l'entité détectable est détecté par un appareil de cytométrie en flux ou un dispositif de balayage à barrettes, éventuellement dans lequel le signal est quantifié.

16. Procédé selon l'une quelconque des revendications précédentes, dans lequel la pluralité de sondes de capture et de recapture comprend de multiples sondes spécifiques pour de multiples acides nucléiques cibles, les multiples sondes étant associées à de multiples particules, chaque particule comprenant des sondes spécifiques pour le même acide nucléique cible, éventuellement
(i) chaque particule étant codée pour fournir l'identité des sondes spécifiques qui y sont présentes, chaque particule étant codée grâce à l'incorporation d'un ou plusieurs fluorophores ayant des caractéristiques spectrales connues ; ou
(ii) les multiples sondes étant situées sur de multiples régions distinctes de la particule.

17. Procédé selon l'une quelconque des revendications précédentes, dans lequel chaque acide nucléique cible est présent à une faible abondance dans l'échantillon, éventuellement dans lequel chaque acide nucléique cible représente moins de 1 %, ou moins de 0,1 %, moins de 1 sur un million ou moins de 1 sur 10 millions des acides nucléiques totaux présents dans l'échantillon biologique.

18. Méthode de diagnostic comprenant une étape consistant à détecter un ou plusieurs acides nucléiques cibles selon l'une quelconque des revendications précédentes.


### SEQUENCE LISTING

<110> Firefly Bioworks, Inc.
<120> Multiplexed PCR with Hydrogel Capture Particles
<130> 2009677-0066
<150> US 61/816,070
   <151> 2013-04-25
<150> US 61/936,826
   <151> 2014-02-06
<160> 20
<170> PatentIn version 3.5
<210> 1
   <211> 31
   <212> RNA
   <213> Artificial Sequence
<220>
   <223> adapter
<400> 1
   gcuaguccua ugcaauguca uaaauauaaa u 31
<210> 2
   <211> 25
   <212> DNA
   <213> Artificial Sequence
<220>
   <223> adapter
<220>
   <221> misc_feature
   <222> (1)..(25)
   <223> combined DNA/RNA oligonucleotide
<400> 2
   cctatgcaat gtcauaaaua uaaau 25
<210> 3
   <211> 28
   <212> DNA
   <213> Artificial Sequence
<220>
   <223> adapter
<220>
   <221> misc_feature
   <222> (1)..(28)
   <223> combined DNA/RNA oligonucleotide
<400> 3
   ggctgagtgc agtgcgagaa auauaaau 28
<210> 4
   <211> 33
   <212> DNA
   <213> Artificial Sequence
<220>
   <223> adapter
<220>
   <221> misc_feature
   <222> (1)..(33)
   <223> combined DNA/RNA oligonucleotide
<400> 4
   ggttggccac gtgacttgat cttaaauaua aau 33
<210> 5
   <211> 34
   <212> DNA
   <213> Artificial Sequence
<220>
   <223> adapter
<220>
   <221> misc_feature
   <222> (1)..(1)
   <223> 5' phosphate
<400> 5
   taataaaata tatccgtcga taagcggatc tatc 34
<210> 6
   <211> 26
   <212> DNA
   <213> Artificial Sequence
<220>
   <223> adapter
<220>
   <221> misc_feature
   <222> (1)..(1)
   <223> 5' phosphate
<400> 6
   taataaaata tatccgtcga taagcg 26
<210> 7
   <211> 25
   <212> DNA
   <213> Artificial Sequence
<220>
   <223> adapter
<220>
   <221> misc_feature
   <222> (1)..(1)
   <223> 5' phosphate
<400> 7
   tttaaaatat atccgtcgat aagcg 25
<210> 8
   <211> 26
   <212> DNA
   <213> Artificial Sequence
<220>
   <223> adapter
<220>
   <221> misc_feature
   <222> (1)..(1)
   <223> 5' phosphate
<400> 8
   taataaaata tatccgtcga taagcg 26
<210> 9
   <211> 31
   <212> DNA
   <213> Artificial Sequence
<220>
   <223> adapter
<220>
   <221> misc_feature
   <222> (1)..(1)
   <223> 5' phosphate
<400> 9
   tttaaaatat atcaagcgtc aattagcgcg a 31
<210> 10
   <211> 21
   <212> DNA
   <213> Artificial Sequence
<220>
   <223> primer
<220>
   <221> misc_feature
   <222> (1)..(1)
   <223> 5' Cy5
<400> 10
   gctagtccta tgcaatgtca t 21
<210> 11
   <211> 24
   <212> DNA
   <213> Artificial Sequence
<220>
   <223> primer
<220>
   <221> misc_feature
   <222> (1)..(1)
   <223> 5' Cy5
<400> 11
   gctagtccta tgcaatgtca taaa 24
<210> 12
   <211> 25
   <212> DNA
   <213> Artificial Sequence
<220>
   <223> primer
<220>
   <221> misc_feature
   <222> (1)..(1)
   <223> 5' Cy5
<400> 12
   cctatgcaat gtcataaata taaat 25
<210> 13
   <211> 21
   <212> DNA
   <213> Artificial Sequence
<220>
   <223> primer
<220>
   <221> misc_feature
   <222> (1)..(1)
   <223> 5' Cy5
<400> 13
   ggctgagtgc agtgcgagaa a 21
<210> 14
   <211> 23
   <212> DNA
   <213> Artificial Sequence
<220>
   <223> primer
<220>
   <221> misc_feature
   <222> (1)..(1)
   <223> 5' Cy5
<400> 14
   ggttggccac gtgacttgat ctt 23
<210> 15
   <211> 20
   <212> DNA
   <213> Artificial Sequence
<220>
   <223> primer
<400> 15
   gatagatccg cttatcgacg 20
<210> 16
   <211> 23
   <212> DNA
   <213> Artificial Sequence
<220>
   <223> primer
<400> 16
   gatagatccg cttatcgacg gat 23
<210> 17
   <211> 26
   <212> DNA
   <213> Artificial Sequence
<220>
   <223> primer
<400> 17
   cgcttatcga cggatatatt ttatta 26
<210> 18
   <211> 25
   <212> DNA
   <213> Artificial Sequence
<220>
   <223> primer
<400> 18
   cgcttatcga cggatatatt ttaaa 25
<210> 19
   <211> 26
   <212> DNA
   <213> Artificial Sequence
<220>
   <223> primer
<400> 19
   cgcttatcga cggatatatt ttatta 26
<210> 20
   <211> 18
   <212> DNA
   <213> Artificial Sequence
<220>
   <223> primer
<400> 20
   tcgcgctaat tgacgctt 18